# EUROPEAN PATENT SPECIFICATION

(11) **EP 3 586 784 B1**
(45) Date of publication and mention of the grant of the patent: **07.02.2024**
(21) Application number: 19182788.0
(22) Date of filing: 27.06.2019
(51) Int. Cl.: A61B 34/30, A61F 2/30, A61B 34/10

(54) **METHODS OF ADJUSTING A VIRTUAL IMPLANT AND RELATED SURGICAL NAVIGATION SYSTEMS**
VERFAHREN ZUR EINSTELLUNG EINES VIRTUELLEN IMPLANTATS UND ZUGEHÖRIGE CHIRURGISCHE NAVIGATIONSSYSTEME
PROCÉDÉS DE RÉGLAGE D'UN IMPLANT VIRTUEL ET SYSTÈMES DE NAVIGATION CHIRURGICALE CORRESPONDANTS

(30) Priority: 27.06.2018 US 201816020302
(43) Date of publication of application: 01.01.2020
(73) Proprietor: Globus Medical, Inc., Audubon, PA 19403 (US)
(72) Inventor: DECKER, Ryan, Pittsburgh, PA 15288 (US); FORSYTH, Jeffrey, Cranston, RI 02921 (US); CRAWFORD, Neil, Chandler, AZ 85224 (US); JOHNSON, Norbert, North Andover, MA 01845 (US); HUSSAIN, Mir, Downingtown, PA 19335 (US); BERKOWITZ, Andrew, Philadelphia, PA 19147 (US); BRAUCKMANN, Michael, Woburn, MA 01801 (US)
(74) Representative: Morabito, Sara

(56) References cited:
- US-A1- 2018 008 355
- US-B2- 9 636 185

## Description

### FIELD

The present disclosure relates to medical devices, and more particularly, surgical navigation systems and related methods and devices.

### BACKGROUND

Medical imaging systems may be used to generate images of a three-dimensional (3D) volume of a body. Position recognition systems can be used to determine the position of and track a particular object in 3D). In robot assisted surgeries, for example, certain objects, such as surgical instruments, may need to be tracked with a high degree of precision as the instrument is being positioned and moved by a robot or by a physician, for example.

Infrared signal based position recognition systems may use passive and/or active sensors or markers to track the objects. In passive sensors or markers, objects to be tracked may include passive sensors, such as reflective spherical balls, which are positioned at strategic locations on the object to be tracked. Infrared transmitters transmit a signal, and the reflective spherical balls reflect the signal to aid in determining the position of the object in 3D. In active sensors or markers, the objects to be tracked include active infrared transmitters, such as light emitting diodes (LEDs), and thus generate their own infrared signals for 3D detection. With either active or passive tracking sensors, the system then geometrically resolves the 3D position of the active and/or passive sensors based on information from or with respect to one or more of the infrared cameras, digital signals, known locations of the active or passive sensors, distance, the time it took to receive the responsive signals, other known variables, or a combination thereof.

These surgical systems can therefore utilize position feedback to precisely guide movement of robotic arms and tools relative to a patients' surgical site. However, these systems lack an ability to virtually plan and prepare for the placement of some tools and implants prior to the surgery. Document US2018008355 A1 relates to surgical robots and associated planning and guidance systems and, more particularly, to a planning arrangement for a guided surgical robot system used, for example, in dental surgery, wherein the planning arrangement is configured to implement a virtual object in a virtual environment, wherein the virtual object is responsive to physical manipulation of a corresponding physical object, in the same manner as the manipulated physical object, in planning and practicing the surgical procedure.

Document D2 discloses the use of a haptic device for control of an object. SUMMARY The inventive method of operating an image-guided surgical system is defined in appended claim 1. The corresponding inventive surgical system is defined in appended claim 14.

### DESCRIPTION OF THE DRAWINGS

The accompanying drawings, which are included to provide a further understanding of the disclosure and constitute a part of this application, illustrate certain non-limiting embodiments of inventive concepts. In the drawings:
FIG. 1 is an overhead view of a potential arrangement for locations of the robotic system, patient, doctor, and other medical personnel during a medical procedure;
FIG. 2 illustrates the robotic system including positioning of the medical robot and the camera relative to the patient according to some embodiments;
FIG. 3 illustrates a medical robotic system in accordance with some embodiments;
FIG. 4 illustrates a portion of a medical robot in accordance with some embodiments;
FIG. 5 illustrates a block diagram of a medical robot in accordance with some embodiments;
FIG. 6 illustrates a medical robot in accordance with some embodiments;
FIGS. 7A-7C illustrate an end-effector in accordance with some embodiments;
FIG. 8 illustrates a medical instrument and the end-effector, before and after, inserting the medical instrument into the guide tube of the end-effector according to some embodiments;
FIGS. 9A-9C illustrate portions of an end-effector and robot arm in accordance with some embodiments;
FIG. 10 illustrates a dynamic reference array, an imaging array, and other components in accordance with some embodiments;
FIG. 11 illustrates a method of registration in accordance with some embodiments;
FIGS. 12A-12B illustrate embodiments of imaging devices according to some embodiments;
FIG. 13A illustrates a portion of a robot including the robot arm and an end-effector in accordance with some embodiments;
FIG. 13B is a close-up view of the end-effector, with a plurality of tracking markers rigidly affixed thereon, shown in FIG. 13A;
FIG. 13C is a tool or instrument with a plurality of tracking markers rigidly affixed thereon according to some embodiments;
FIG. 14A is an alternative version of an end-effector with moveable tracking markers in a first configuration;
FIG. 14B is the end-effector shown in FIG. 14A with the moveable tracking markers in a second configuration;
FIG. 14C shows the template of tracking markers in the first configuration from FIG. 14A;
FIG. 14D shows the template of tracking markers in the second configuration from FIG. 14B;
FIG. 15A shows an alternative version of the end-effector having only a single tracking marker affixed thereto;
FIG. 15B shows the end-effector of FIG. 15A with an instrument disposed through the guide tube;
FIG. 15C shows the end-effector of FIG. 15A with the instrument in two different positions, and the resulting logic to determine if the instrument is positioned within the guide tube or outside of the guide tube;
FIG. 15D shows the end-effector of FIG. 15A with the instrument in the guide tube at two different frames and its relative distance to the single tracking marker on the guide tube;
FIG. 15E shows the end-effector of FIG. 15A relative to a coordinate system;
FIG. 16 is a block diagram of a method for navigating and moving the end-effector of the robot to a desired target trajectory;
FIGS. 17A-17B depict an instrument for inserting an expandable implant having fixed and moveable tracking markers in contracted and expanded positions, respectively;
FIGS. 18A-18B depict an instrument for inserting an articulating implant having fixed and moveable tracking markers in insertion and angled positions, respectively;
FIGS. 19A depicts an example of a robot with interchangeable or alternative end-effectors;
FIG. 19B depicts an example of a robot with an instrument style end-effector coupled thereto;
FIG. 20 is a block diagram illustrating a controller according to some embodiments;
FIG. 21 illustrates a tracking system detecting a probe and determining a placement of a virtual implant according to the invention;
FIG. 22 is a flow chart illustrating example operations of a surgical robotic navigation system according to the invention; and
FIG. 23A-B are examples of a telescoping probe according to some examples.
FIGS. 24A-B, 25 and 26 depict examples of movements of a probe that may be detected by a tracking system and used to adjust a placement of a virtual implant according to some examples;

### DETAILED DESCRIPTION

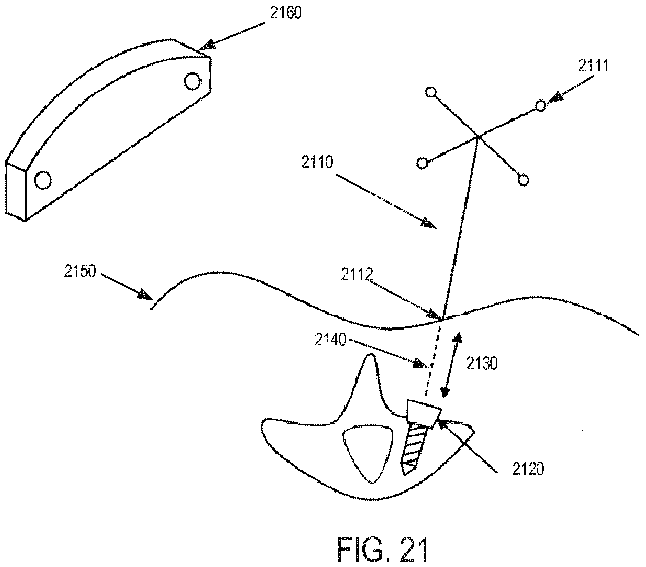

Embodiments of the invention are described in fig.21-26 together with the corresponding paragraphs.

The teachings of the present disclosure may be used and practiced in other embodiments and practiced or carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

The following discussion is presented to enable a person skilled in the art to make and use embodiments of the present disclosure. Various modifications to the illustrated embodiments will be readily apparent to those skilled in the art, and the principles herein can be applied to other embodiments and applications without departing from embodiments of the present disclosure. The following detailed description is to be read with reference to the figures, in which like elements in different figures have like reference numerals.

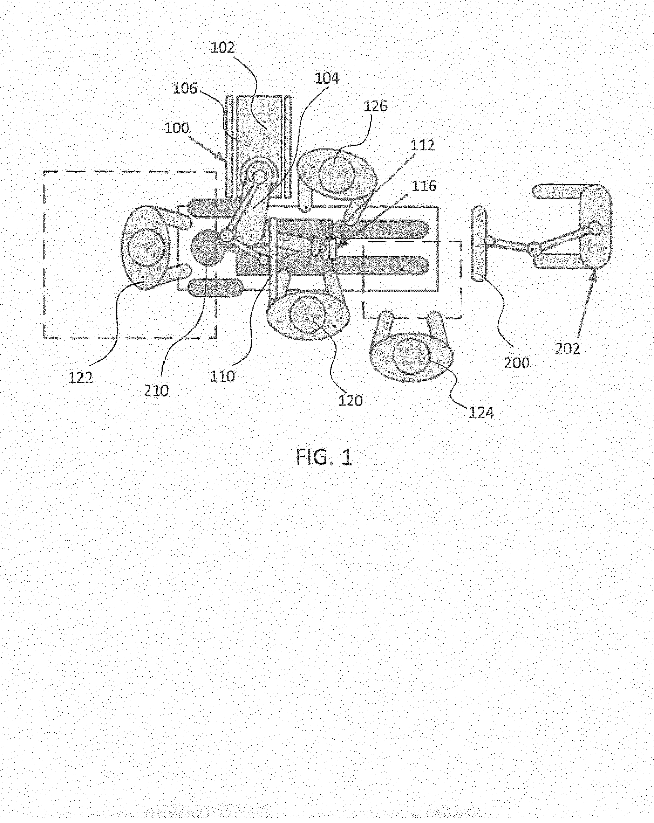

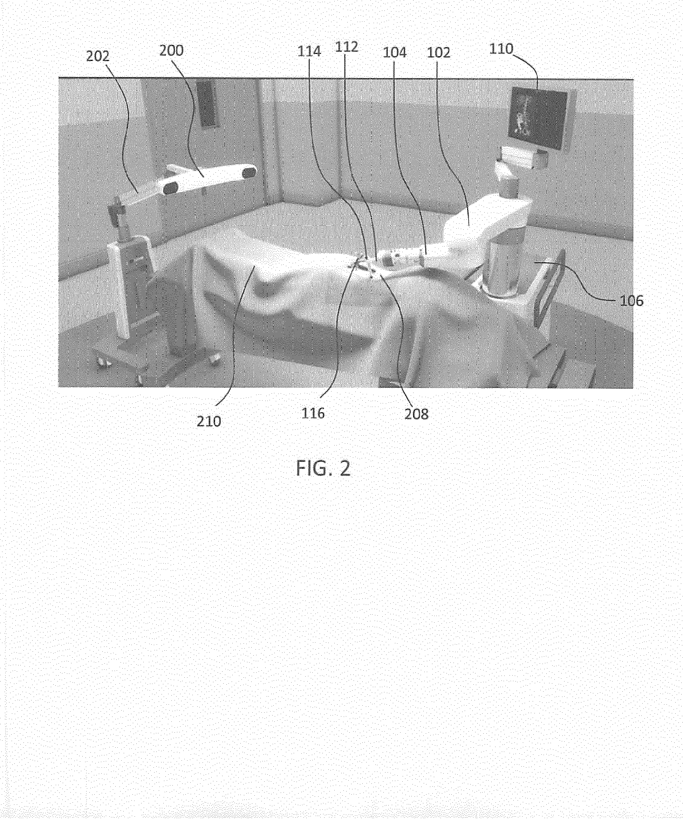

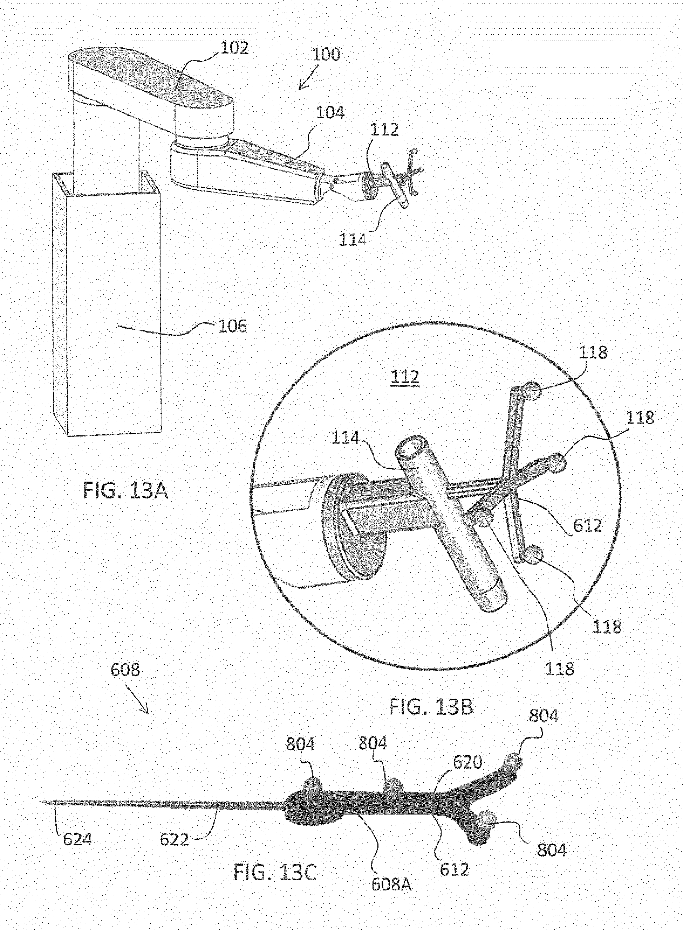

Turning now to the drawings, FIGS. 1 and 2 illustrate a medical robot system 100 in accordance with some embodiments. Medical robot system 100 may include, for example, a medical robot 102, one or more robot arms 104, a base 106, a display 110, an end-effector 112, for example, including a guide tube 114, and one or more tracking markers 118. The medical robot system 100 may include a patient tracking device 116 also including one or more tracking markers 118, which is adapted to be secured directly to the patient 210 (e.g., to a bone of the patient 210). The medical robot system 100 may also use a camera(s) 200, for example, positioned on a camera stand 202. The camera stand 202 can have any suitable configuration to move, orient, and support the camera 200 in a desired position. The camera 200 may include any suitable camera or cameras, such as one or more infrared cameras (e.g., bifocal or stereophotogrammetric cameras), able to identify, for example, active and passive tracking markers 118 (shown as part of patient tracking device 116 in FIG. 2 and shown by enlarged view in FIGS. 13A-13B) in a given measurement volume viewable from the perspective of the camera 200. The camera 200 may scan the given measurement volume and detect the light that comes from the markers 118 in order to identify and determine the position of the markers 118 in three-dimensions. For example, active markers 118 may include infrared-emitting markers that are activated by an electrical signal (e.g., infrared light emitting diodes (LEDs)), and/or passive markers 118 may include retro-reflective markers that reflect infrared light (e.g., they reflect incoming IR radiation into the direction of the incoming light), for example, emitted by illuminators on the camera 200 or other suitable device.

FIGS. 1 and 2 illustrate a potential configuration for the placement of the medical robot system 100 in an operating room environment. For example, the robot 102 may be positioned near or next to patient 210. Although depicted near the head of the patient 210, it will be appreciated that the robot 102 can be positioned at any suitable location near the patient 210 depending on the area of the patient 210 undergoing the operation. The camera 200 may be separated from the robot system 100 and positioned at the foot of patient 210. This location allows the camera 200 to have a direct visual line of sight to the medical field 208. Again, it is contemplated that the camera 200 may be located at any suitable position having line of sight to the medical field 208. In the configuration shown, the doctor 120 may be positioned across from the robot 102, but is still able to manipulate the end-effector 112 and the display 110. A medical assistant 126 may be positioned across from the doctor 120 again with access to both the end-effector 112 and the display 110. If desired, the locations of the doctor 120 and the assistant 126 may be reversed. The traditional areas for the anesthesiologist 122 and the nurse or scrub tech 124 may remain unimpeded by the locations of the robot 102 and camera 200.

With respect to the other components of the robot 102, the display 110 can be attached to the medical robot 102 and in some embodiments, display 110 can be detached from medical robot 102, either within a surgical room (or other medical facility) with the medical robot 102, or in a remote location. End-effector 112 may be coupled to the robot arm 104 and controlled by at least one motor. In some embodiments, end-effector 112 can comprise a guide tube 114, which is able to receive and orient a medical instrument 608 (described further herein) used on the patient 210. As used herein, the term "end-effector" is used interchangeably with the terms "end-effectuator" and "effectuator element." Although generally shown with a guide tube 114, it will be appreciated that the end-effector 112 may be replaced with any suitable instrumentation suitable for use in surgery. In some embodiments, end-effector 112 can comprise any known structure for effecting the movement of the medical instrument 608 in a desired manner.

The medical robot 102 is able to control the translation and orientation of the end-effector 112. The robot 102 is able to move end-effector 112 along x-, y-, and z-axes, for example. The end-effector 112 can be configured for selective rotation about one or more of the x-, y-, and z-axis, and a Z Frame axis (such that one or more of the Euler Angles (e.g., roll, pitch, and/or yaw) associated with end-effector 112 can be selectively controlled). In some embodiments, selective control of the translation and orientation of end-effector 112 can permit performance of medical procedures with significantly improved accuracy compared to conventional robots that use, for example, a six degree of freedom robot arm comprising only rotational axes. For example, the medical robot system 100 may be used to provide medical imaging and/or to operate on patient 210, and robot arm 104 can be positioned above the body of patient 210, with end-effector 112 selectively angled relative to the z-axis toward the body of patient 210.

In some embodiments, the position of the medical instrument 608 can be dynamically updated so that medical robot 102 can be aware of the location of the medical instrument 608 at all times during the procedure. Consequently, in some embodiments, medical robot 102 can move the medical instrument 608 to the desired position quickly without any further assistance from a physician (unless the physician so desires). In some further embodiments, medical robot 102 can be configured to correct the path of the medical instrument 608 if the medical instrument 608 strays from the selected, preplanned trajectory. In some embodiments, medical robot 102 can be configured to permit stoppage, modification, and/or manual control of the movement of end-effector 112 and/or the medical instrument 608. Thus, in use, in some embodiments, a physician or other user can operate the system 100, and has the option to stop, modify, or manually control the autonomous movement of end-effector 112 and/or the medical instrument 608. Further details of medical robot system 100 including the control and movement of a medical instrument 608 by medical robot 102 can be found in co-pending U.S. patent application Ser. No. 13/924,505, which is incorporated herein by reference in its entirety.

The robotic medical system 100 can comprise one or more tracking markers 118 configured to track the movement of robot arm 104, end-effector 112, patient 210, and/or the medical instrument 608 in three dimensions. In some embodiments, a plurality of tracking markers 118 can be mounted (or otherwise secured) thereon to an outer surface of the robot 102, such as, for example and without limitation, on base 106 of robot 102, on robot arm 104, and/or on the end-effector 112. In some embodiments, at least one tracking marker 118 of the plurality of tracking markers 118 can be mounted or otherwise secured to the end-effector 112. One or more tracking markers 118 can further be mounted (or otherwise secured) to the patient 210. In some embodiments, the plurality of tracking markers 118 can be positioned on the patient 210 spaced apart from the medical field 208 to reduce the likelihood of being obscured by the doctor, medical tools, or other parts of the robot 102. Further, one or more tracking markers 118 can be further mounted (or otherwise secured) to the medical tools 608 (e.g., an ultrasound transducer, a screw driver, dilator, implant inserter, or the like). Thus, the tracking markers 118 enable each of the marked objects (e.g., the end-effector 112, the patient 210, and the medical tools 608) to be tracked by the robot 102. In some embodiments, system 100 can use tracking information collected from each of the marked objects to calculate the orientation and location, for example, of the end-effector 112, the medical instrument 608 (e.g., positioned in the tube 114 of the end-effector 112), and the relative position of the patient 210.

The markers 118 may include radiopaque or optical markers. The markers 118 may be suitably shaped include spherical, spheroid, cylindrical, cube, cuboid, or the like. In some embodiments, one or more of markers 118 may be optical markers. In some embodiments, the positioning of one or more tracking markers 118 on end-effector 112 can increase/maximize the accuracy of the positional measurements by serving to check or verify the position of end-effector 112. Further details of medical robot system 100 including the control, movement and tracking of medical robot 102 and of a medical instrument 608 can be found in U.S. patent publication No. 2016/0242849, which is incorporated herein by reference in its entirety.

Some embodiments include one or more markers 118 coupled to the medical instrument 608. In some embodiments, these markers 118, for example, coupled to the patient 210 and medical instruments 608, as well as markers 118 coupled to the end-effector 112 of the robot 102 can comprise conventional infrared light-emitting diodes (LEDs) or an Optotrak^{®} diode capable of being tracked using a commercially available infrared optical tracking system such as Optotrak^{®}. Optotrak^{®} is a registered trademark of Northern Digital Inc., Waterloo, Ontario, Canada. In other embodiments, markers 118 can comprise conventional reflective spheres capable of being tracked using a commercially available optical tracking system such as Polaris Spectra. Polaris Spectra is also a registered trademark of Northern Digital, Inc. In some embodiments, the markers 118 coupled to the end-effector 112 are active markers which comprise infrared light-emitting diodes which may be turned on and off, and the markers 118 coupled to the patient 210 and the medical instruments 608 comprise passive reflective spheres.

In some embodiments, light emitted from and/or reflected by markers 118 can be detected by camera 200 and can be used to monitor the location and movement of the marked objects. In alternative embodiments, markers 118 can comprise a radio-frequency and/or electromagnetic reflector or transceiver and the camera 200 can include or be replaced by a radio-frequency and/or electromagnetic transceiver.

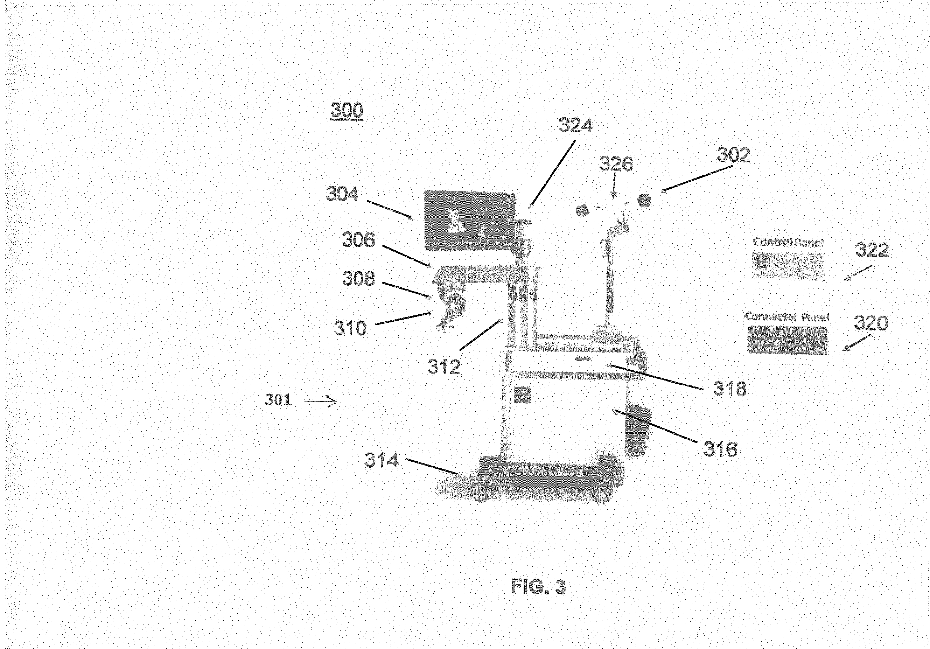

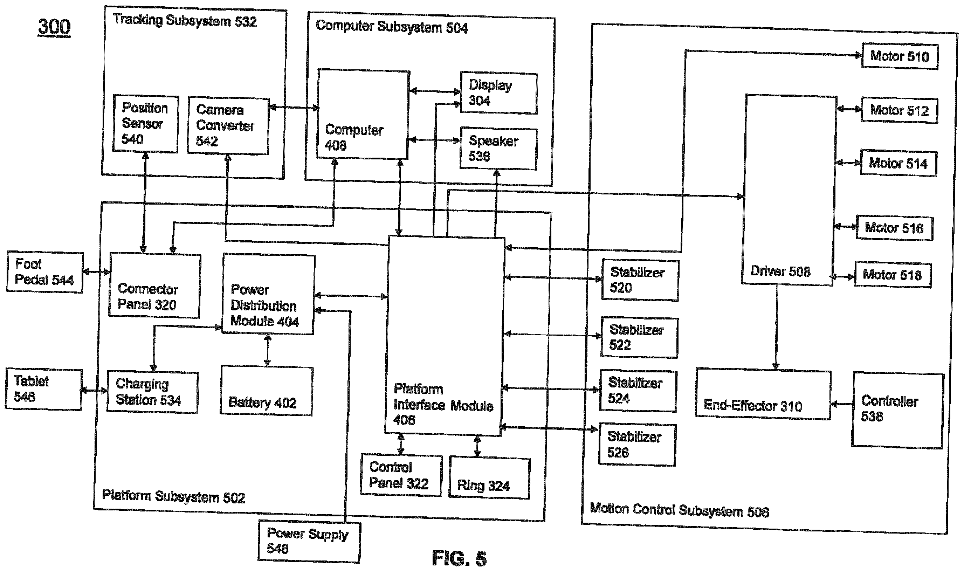

Similar to medical robot system 100, FIG. 3 illustrates a medical robot system 300 and camera stand 302, in a docked configuration, consistent with some embodiments of the present disclosure. Medical robot system 300 may comprise a robot 301 including a display 304, upper arm 306, lower arm 308, end-effector 310, vertical column 312, casters 314, cabinet 316, tablet drawer 318, connector panel 320, control panel 322, and ring of information 324. Camera stand 302 may comprise camera 326. These components are described in greater with respect to FIG. 5. FIG. 3 illustrates the medical robot system 300 in a docked configuration where the camera stand 302 is nested with the robot 301, for example, when not in use. It will be appreciated by those skilled in the art that the camera 326 and robot 301 may be separated from one another and positioned at any appropriate location during the medical procedure, for example, as shown in FIGS. 1 and 2.

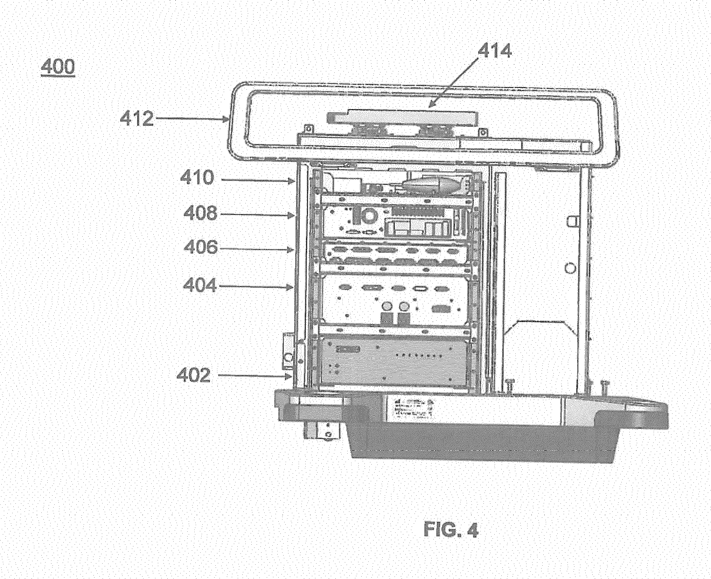

FIG. 4 illustrates a base 400 consistent with some embodiments of the present disclosure. Base 400 may be a portion of medical robot system 300 and comprise cabinet 316. Cabinet 316 may house certain components of medical robot system 300 including but not limited to a battery 402, a power distribution module 404, a platform interface board module 406, a computer 408, a handle 412, and a tablet drawer 414. The connections and relationship between these components is described in greater detail with respect to FIG. 5.

FIG. 5 illustrates a block diagram of certain components of some embodiments of medical robot system 300. Medical robot system 300 may comprise platform subsystem 502, computer subsystem 504, motion control subsystem 506, and tracking subsystem 532. Platform subsystem 502 may further comprise battery 402, power distribution module 404, platform interface board module 406, and tablet charging station 534. Computer subsystem 504 may further comprise computer 408, display 304, and speaker 536. Motion control subsystem 506 may further comprise driver circuit 508, motors 510, 512, 514, 516, 518, stabilizers 520, 522, 524, 526, end-effector 310, and controller 538. Tracking subsystem 532 may further comprise position sensor 540 and camera converter 542. System 300 may also comprise a foot pedal 544 and tablet 546.

Input power is supplied to system 300 via a power source 548 which may be provided to power distribution module 404. Power distribution module 404 receives input power and is configured to generate different power supply voltages that are provided to other modules, components, and subsystems of system 300. Power distribution module 404 may be configured to provide different voltage supplies to platform interface module 406, which may be provided to other components such as computer 408, display 304, speaker 536, driver 508 to, for example, power motors 512, 514, 516, 518 and end-effector 310, motor 510, ring 324, camera converter 542, and other components for system 300 for example, fans for cooling the electrical components within cabinet 316.

Power distribution module 404 may also provide power to other components such as tablet charging station 534 that may be located within tablet drawer 318. Tablet charging station 534 may be in wireless or wired communication with tablet 546 for charging table 546. Tablet 546 may be used by a doctor consistent with the present disclosure and described herein.

Power distribution module 404 may also be connected to battery 402, which serves as temporary power source in the event that power distribution module 404 does not receive power from input power 548. At other times, power distribution module 404 may serve to charge battery 402 if necessary.

Other components of platform subsystem 502 may also include connector panel 320, control panel 322, and ring 324. Connector panel 320 may serve to connect different devices and components to system 300 and/or associated components and modules. Connector panel 320 may contain one or more ports that receive lines or connections from different components. For example, connector panel 320 may have a ground terminal port that may ground system 300 to other equipment, a port to connect foot pedal 544 to system 300, a port to connect to tracking subsystem 532, which may comprise position sensor 540, camera converter 542, and cameras 326 associated with camera stand 302. Connector panel 320 may also include other ports to allow USB, Ethernet, HDMI communications to other components, such as computer 408.

Control panel 322 may provide various buttons or indicators that control operation of system 300 and/or provide information regarding system 300. For example, control panel 322 may include buttons to power on or off system 300, lift or lower vertical column 312, and lift or lower stabilizers 520-526 that may be designed to engage casters 314 to lock system 300 from physically moving. Other buttons may stop system 300 in the event of an emergency, which may remove all motor power and apply mechanical brakes to stop all motion from occurring. Control panel 322 may also have indicators notifying the user of certain system conditions such as a line power indicator or status of charge for battery 402.

Ring 324 may be a visual indicator to notify the user of system 300 of different modes that system 300 is operating under and certain warnings to the user.

Computer subsystem 504 includes computer 408, display 304, and speaker 536. Computer 504 includes an operating system and software to operate system 300. Computer 504 may receive and process information from other components (for example, tracking subsystem 532, platform subsystem 502, and/or motion control subsystem 506) in order to display information to the user. Further, computer subsystem 504 may also include speaker 536 to provide audio to the user.

Tracking subsystem 532 may include position sensor 504 and converter 542. Tracking subsystem 532 may correspond to camera stand 302 including camera 326 as described with respect to FIG. 3. Position sensor 504 may be camera 326. Tracking subsystem may track the location of certain markers that are located on the different components of system 300 and/or instruments used by a user during a medical procedure. This tracking may be conducted in a manner consistent with the present disclosure including the use of infrared technology that tracks the location of active or passive elements, such as LEDs or reflective markers, respectively. The location, orientation, and position of structures having these types of markers may be provided to computer 408 which may be shown to a user on display 304. For example, a medical instrument 608 having these types of markers and tracked in this manner (which may be referred to as a navigational space) may be shown to a user in relation to a three dimensional image of a patient's anatomical structure.

Motion control subsystem 506 may be configured to physically move vertical column 312, upper arm 306, lower arm 308, or rotate end-effector 310. The physical movement may be conducted through the use of one or more motors 510-518. For example, motor 510 may be configured to vertically lift or lower vertical column 312. Motor 512 may be configured to laterally move upper arm 308 around a point of engagement with vertical column 312 as shown in FIG. 3. Motor 514 may be configured to laterally move lower arm 308 around a point of engagement with upper arm 308 as shown in FIG. 3. Motors 516 and 518 may be configured to move end-effector 310 in a manner such that one may control the roll and one may control the tilt, thereby providing multiple angles that end-effector 310 may be moved. These movements may be achieved by controller 538 which may control these movements through load cells disposed on end-effector 310 and activated by a user engaging these load cells to move system 300 in a desired manner.

Moreover, system 300 may provide for automatic movement of vertical column 312, upper arm 306, and lower arm 308 through a user indicating on display 304 (which may be a touchscreen input device) the location of a medical instrument or component on a three dimensional image of the patient's anatomy on display 304. The user may initiate this automatic movement by stepping on foot pedal 544 or some other input means.

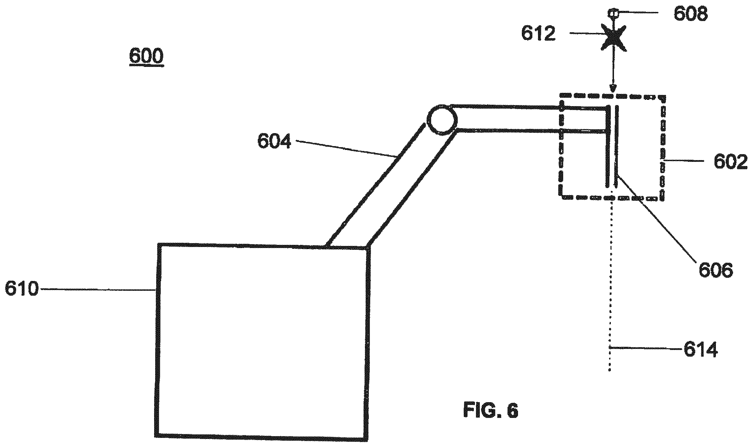

FIG. 6 illustrates a medical robot system 600 consistent with some embodiments. Medical robot system 600 may comprise end-effector 602, robot arm 604, guide tube 606, instrument 608, and robot base 610. Instrument tool 608 may be attached to a tracking array 612 including one or more tracking markers (such as markers 118) and have an associated trajectory 614. Trajectory 614 may represent a path of movement that instrument tool 608 is configured to travel once it is positioned through or secured in guide tube 606, for example, a path of insertion of instrument tool 608 into a patient. In an operation, robot base 610 may be configured to be in electronic communication with robot arm 604 and end-effector 602 so that medical robot system 600 may assist a user (for example, a doctor) in operating on the patient 210. Medical robot system 600 may be consistent with previously described medical robot system 100 and 300.

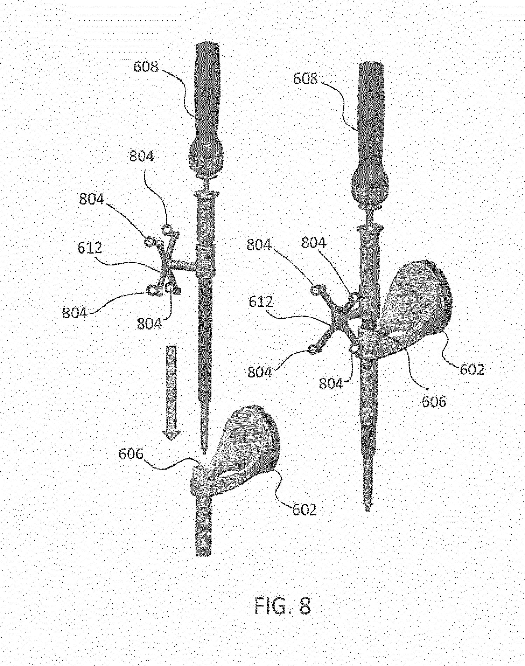

A tracking array 612 may be mounted on instrument 608 to monitor the location and orientation of instrument tool 608. The tracking array 612 may be attached to an instrument 608 and may comprise tracking markers 804. As best seen in FIG. 8, tracking markers 804 may be, for example, light emitting diodes and/or other types of reflective markers (e.g., markers 118 as described elsewhere herein). The tracking devices may be one or more line of sight devices associated with the medical robot system. As an example, the tracking devices may be one or more cameras 200, 326 associated with the medical robot system 100, 300 and may also track tracking array 612 for a defined domain or relative orientations of the instrument 608 in relation to the robot arm 604, the robot base 610, end-effector 602, and/or the patient 210. The tracking devices may be consistent with those structures described in connection with camera stand 302 and tracking subsystem 532.

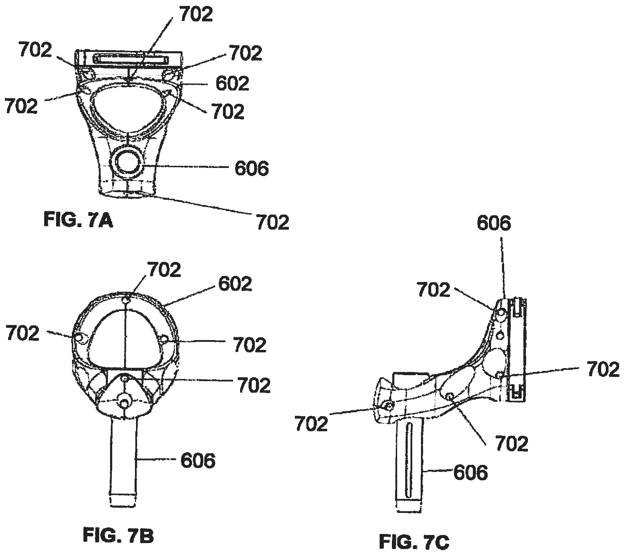

FIGS. 7A, 7B, and 7C illustrate a top view, front view, and side view, respectively, of end-effector 602 consistent with some embodiments. End-effector 602 may comprise one or more tracking markers 702. Tracking markers 702 may be light emitting diodes or other types of active and passive markers, such as tracking markers 118 that have been previously described. In some embodiments, the tracking markers 702 are active infrared-emitting markers that are activated by an electrical signal (e.g., infrared light emitting diodes (LEDs)). Thus, tracking markers 702 may be activated such that the infrared markers 702 are visible to the camera 200, 326 or may be deactivated such that the infrared markers 702 are not visible to the camera 200, 326. Thus, when the markers 702 are active, the end-effector 602 may be controlled by the system 100, 300, 600, and when the markers 702 are deactivated, the end-effector 602 may be locked in position and unable to be moved by the system 100, 300, 600.

Markers 702 may be disposed on or within end-effector 602 in a manner such that the markers 702 are visible by one or more cameras 200, 326 or other tracking devices associated with the medical robot system 100, 300, 600. The camera 200, 326 or other tracking devices may track end-effector 602 as it moves to different positions and viewing angles by following the movement of tracking markers 702. The location of markers 702 and/or end-effector 602 may be shown on a display 110, 304 associated with the medical robot system 100, 300, 600, for example, display 110 as shown in FIG. 2 and/or display 304 shown in FIG. 3. This display 110, 304 may allow a user to ensure that end-effector 602 is in a desirable position in relation to robot arm 604, robot base 610, the patient 210, and/or the user.

For example, as shown in FIG. 7A, markers 702 may be placed around the surface of end-effector 602 so that a tracking device placed away from the medical field 208 and facing toward the robot 102, 301 and the camera 200, 326 is able to view at least 3 of the markers 702 through a range of common orientations of the end-effector 602 relative to the tracking device. For example, distribution of markers 702 in this way allows end-effector 602 to be monitored by the tracking devices when end-effector 602 is translated and rotated in the medical field 208.

In addition, in some embodiments, end-effector 602 may be equipped with infrared (IR) receivers that can detect when an external camera 200, 326 is getting ready to read markers 702. Upon this detection, end-effector 602 may then illuminate markers 702. The detection by the IR receivers that the external camera 200, 326 is ready to read markers 702 may signal the need to synchronize a duty cycle of markers 702, which may be light emitting diodes, to an external camera 200, 326. This may also allow for lower power consumption by the robotic system as a whole, whereby markers 702 would only be illuminated at the appropriate time instead of being illuminated continuously. Further, in some embodiments, markers 702 may be powered off to prevent interference with other navigation tools, such as different types of medical instruments 608.

FIG. 8 depicts one type of medical instrument 608 including a tracking array 612 and tracking markers 804. Tracking markers 804 may be of any type described herein including but not limited to light emitting diodes or reflective spheres. Markers 804 are monitored by tracking devices associated with the medical robot system 100, 300, 600 and may be one or more of the line of sight cameras 200, 326. The cameras 200, 326 may track the location of instrument 608 based on the position and orientation of tracking array 612 and markers 804. A user, such as a doctor 120, may orient instrument 608 in a manner so that tracking array 612 and markers 804 are sufficiently recognized by the tracking device or camera 200, 326 to display instrument 608 and markers 804 on, for example, display 110 of the medical robot system.

The manner in which a doctor 120 may place instrument 608 into guide tube 606 of the end-effector 602 and adjust the instrument 608 is evident in FIG. 8. The hollow tube or guide tube 114, 606 of the end-effector 112, 310, 602 is sized and configured to receive at least a portion of the medical instrument 608. The guide tube 114, 606 is configured to be oriented by the robot arm 104 such that insertion and trajectory for the medical instrument 608 is able to reach a desired anatomical target within or upon the body of the patient 210. The medical instrument 608 may include at least a portion of a generally cylindrical instrument. Although a screw driver is exemplified as the medical tool 608, it will be appreciated that any suitable medical tool 608 may be positioned by the end-effector 602. By way of example, the medical instrument 608 may include one or more of a guide wire, cannula, a retractor, a drill, a reamer, a screw driver, an insertion tool, a removal tool, or the like. Although the hollow tube 114, 606 is generally shown as having a cylindrical configuration, it will be appreciated by those of skill in the art that the guide tube 114, 606 may have any suitable shape, size, and configuration desired to accommodate the medical instrument 608 and access the medical site.

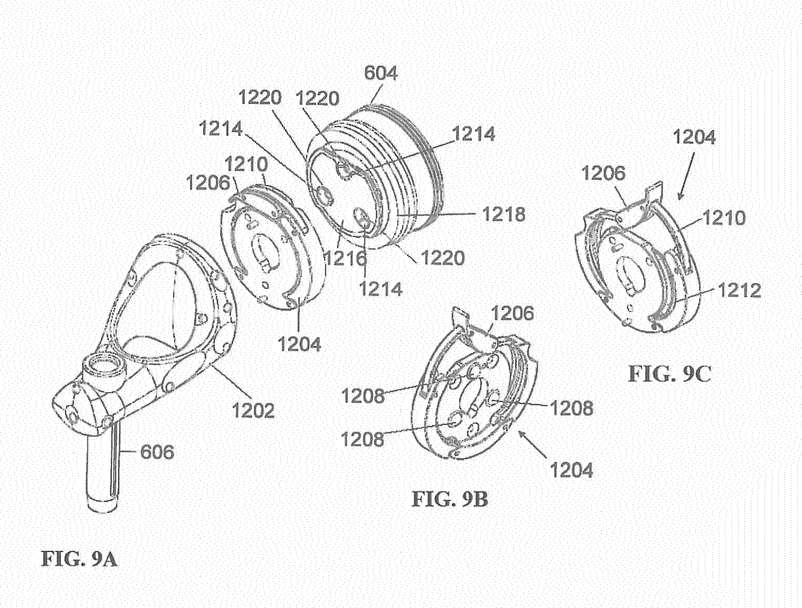

FIGS. 9A-9C illustrate end-effector 602 and a portion of robot arm 604 consistent with some embodiments. End-effector 602 may further comprise body 1202 and clamp 1204. Clamp 1204 may comprise handle 1206, balls 1208, spring 1210, and lip 1212. Robot arm 604 may further comprise depressions 1214, mounting plate 1216, lip 1218, and magnets 1220.

End-effector 602 may mechanically interface and/or engage with the medical robot system and robot arm 604 through one or more couplings. For example, end-effector 602 may engage with robot arm 604 through a locating coupling and/or a reinforcing coupling. Through these couplings, end-effector 602 may fasten with robot arm 604 outside a flexible and sterile barrier. In some embodiments, the locating coupling may be a magnetically kinematic mount and the reinforcing coupling may be a five bar over center clamping linkage.

With respect to the locating coupling, robot arm 604 may comprise mounting plate 1216, which may be non-magnetic material, one or more depressions 1214, lip 1218, and magnets 1220. Magnet 1220 is mounted below each of depressions 1214. Portions of clamp 1204 may comprise magnetic material and be attracted by one or more magnets 1220. Through the magnetic attraction of clamp 1204 and robot arm 604, balls 1208 become seated into respective depressions 1214. For example, balls 1208 as shown in FIG. 9B would be seated in depressions 1214 as shown in FIG. 9A. This seating may be considered a magnetically-assisted kinematic coupling. Magnets 1220 may be configured to be strong enough to support the entire weight of end-effector 602 regardless of the orientation of end-effector 602. The locating coupling may be any style of kinematic mount that uniquely restrains six degrees of freedom.

With respect to the reinforcing coupling, portions of clamp 1204 may be configured to be a fixed ground link and as such clamp 1204 may serve as a five bar linkage. Closing clamp handle 1206 may fasten end-effector 602 to robot arm 604 as lip 1212 and lip 1218 engage clamp 1204 in a manner to secure end-effector 602 and robot arm 604. When clamp handle 1206 is closed, spring 1210 may be stretched or stressed while clamp 1204 is in a locked position. The locked position may be a position that provides for linkage past center. Because of a closed position that is past center, the linkage will not open absent a force applied to clamp handle 1206 to release clamp 1204. Thus, in a locked position end-effector 602 may be robustly secured to robot arm 604.

Spring 1210 may be a curved beam in tension. Spring 1210 may be comprised of a material that exhibits high stiffness and high yield strain such as virgin PEEK (poly-ether-etherketone). The linkage between end-effector 602 and robot arm 604 may provide for a sterile barrier between end-effector 602 and robot arm 604 without impeding fastening of the two couplings.

The reinforcing coupling may be a linkage with multiple spring members. The reinforcing coupling may latch with a cam or friction based mechanism. The reinforcing coupling may also be a sufficiently powerful electromagnet that will support fastening end-effector 102 to robot arm 604. The reinforcing coupling may be a multi-piece collar completely separate from either end-effector 602 and/or robot arm 604 that slips over an interface between end-effector 602 and robot arm 604 and tightens with a screw mechanism, an over center linkage, or a cam mechanism.

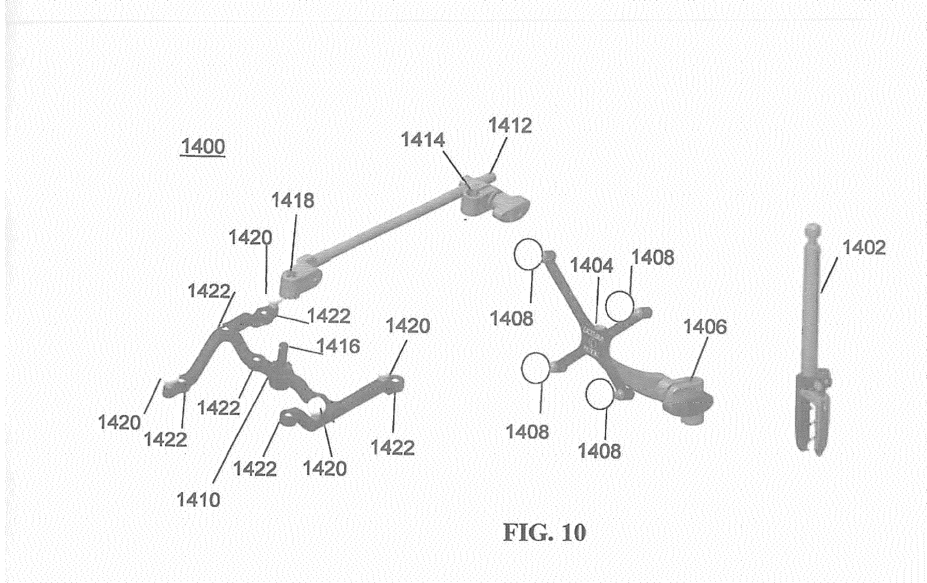

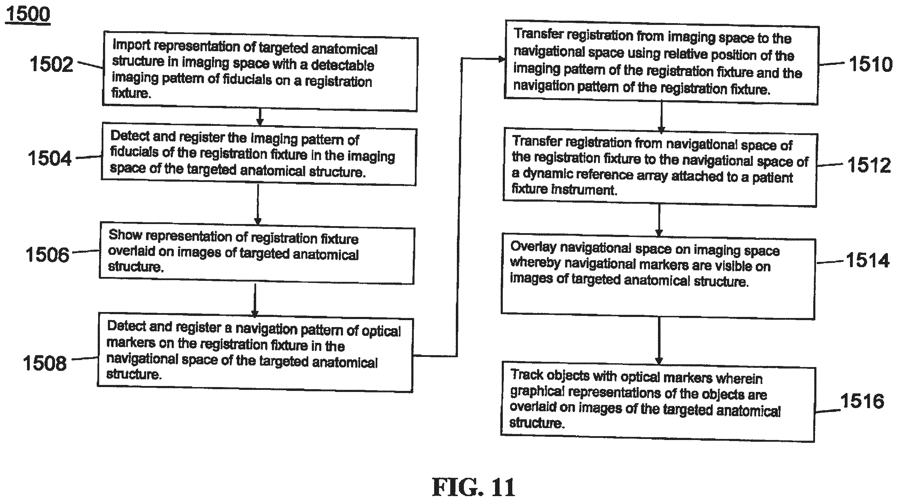

Referring to FIGS. 10 and 11, prior to or during a medical procedure, certain registration procedures may be conducted to track objects and a target anatomical structure of the patient 210 both in a navigation space and an image space. To conduct such registration, a registration system 1400 may be used as illustrated in FIG. 10.

To track the position of the patient 210, a patient tracking device 116 may include a patient fixation instrument 1402 to be secured to a rigid anatomical structure of the patient 210 and a dynamic reference base (DRB) 1404 may be securely attached to the patient fixation instrument 1402. For example, patient fixation instrument 1402 may be inserted into opening 1406 of dynamic reference base 1404. Dynamic reference base 1404 may contain markers 1408 that are visible to tracking devices, such as tracking subsystem 532. These markers 1408 may be optical markers or reflective spheres, such as tracking markers 118, as previously discussed herein.

Patient fixation instrument 1402 is attached to a rigid anatomy of the patient 210 and may remain attached throughout the medical procedure. In some embodiments, patient fixation instrument 1402 is attached to a rigid area of the patient 210, for example, a bone that is located away from the targeted anatomical structure subject to the medical procedure. In order to track the targeted anatomical structure, dynamic reference base 1404 is associated with the targeted anatomical structure through the use of a registration fixture that is temporarily placed on or near the targeted anatomical structure in order to register the dynamic reference base 1404 with the location of the targeted anatomical structure.

A registration fixture 1410 is attached to patient fixation instrument 1402 through the use of a pivot arm 1412. Pivot arm 1412 is attached to patient fixation instrument 1402 by inserting patient fixation instrument 1402 through an opening 1414 of registration fixture 1410. Pivot arm 1412 is attached to registration fixture 1410 by, for example, inserting a knob 1416 through an opening 1418 of pivot arm 1412.

Using pivot arm 1412, registration fixture 1410 may be placed over the targeted anatomical structure and its location may be determined in an image space and navigation space using tracking markers 1420 and/or fiducials 1422 on registration fixture 1410. Registration fixture 1410 may contain a collection of markers 1420 that are visible in a navigational space (for example, markers 1420 may be detectable by tracking subsystem 532). Tracking markers 1420 may be optical markers visible in infrared light as previously described herein. Registration fixture 1410 may also contain a collection of fiducials 1422, for example, such as bearing balls, that are visible in an imaging space (for example, a three dimension CT image). As described in greater detail with respect to FIG. 11, using registration fixture 1410, the targeted anatomical structure may be associated with dynamic reference base 1404 thereby allowing depictions of objects in the navigational space to be overlaid on images of the anatomical structure. Dynamic reference base 1404, located at a position away from the targeted anatomical structure, may become a reference point thereby allowing removal of registration fixture 1410 and/or pivot arm 1412 from the medical area.

FIG. 11 provides a method 1500 for registration consistent with the present disclosure. Method 1500 begins at step 1502 wherein a graphical representation (or image(s)) of the targeted anatomical structure may be imported into system 100, 300 600, for example computer 408. The graphical representation may be three dimensional CT or a fluoroscope scan of the targeted anatomical structure of the patient 210 which includes registration fixture 1410 and a detectable imaging pattern of fiducials 1420.

At step 1504, an imaging pattern of fiducials 1420 is detected and registered in the imaging space and stored in computer 408. Optionally, at this time at step 1506, a graphical representation of the registration fixture 1410 may be overlaid on the images of the targeted anatomical structure.

At step 1508, a navigational pattern of registration fixture 1410 is detected and registered by recognizing markers 1420. Markers 1420 may be optical markers that are recognized in the navigation space through infrared light by tracking subsystem 532 via position sensor 540. Thus, the location, orientation, and other information of the targeted anatomical structure is registered in the navigation space. Therefore, registration fixture 1410 may be recognized in both the image space through the use of fiducials 1422 and the navigation space through the use of markers 1420. At step 1510, the registration of registration fixture 1410 in the image space is transferred to the navigation space. This transferal is done, for example, by using the relative position of the imaging pattern of fiducials 1422 compared to the position of the navigation pattern of markers 1420.

At step 1512, registration of the navigation space of registration fixture 1410 (having been registered with the image space) is further transferred to the navigation space of dynamic registration array 1404 attached to patient fixture instrument 1402. Thus, registration fixture 1410 may be removed and dynamic reference base 1404 may be used to track the targeted anatomical structure in both the navigation and image space because the navigation space is associated with the image space.

At steps 1514 and 1516, the navigation space may be overlaid on the image space and objects with markers visible in the navigation space (for example, medical instruments 608 with optical markers 804). The objects may be tracked through graphical representations of the medical instrument 608 on the images of the targeted anatomical structure.

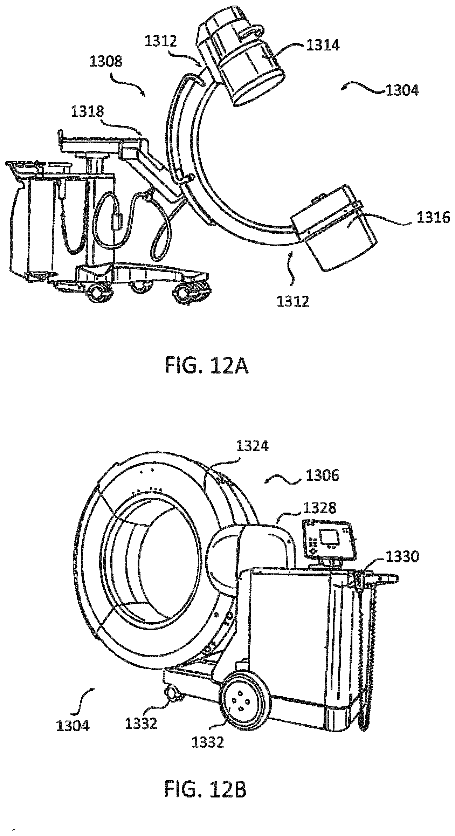

FIGS. 12A-12B illustrate imaging devices 1304 that may be used in conjunction with robot systems 100, 300, 600 to acquire pre-operative, intra-operative, post-operative, and/or real-time image data of patient 210. Any appropriate subject matter may be imaged for any appropriate procedure using the imaging system 1304. The imaging system 1304 may be any imaging device such as imaging device 1306 and/or a C-arm 1308 device. It may be desirable to take x-rays of patient 210 from a number of different positions, without the need for frequent manual repositioning of patient 210 which may be required in an x-ray system. As illustrated in FIG. 12A, the imaging system 1304 may be in the form of a C-arm 1308 that includes an elongated C-shaped member terminating in opposing distal ends 1312 of the "C" shape. C-shaped member 1130 may further comprise an x-ray source 1314 and an image receptor 1316. The space within C-arm 1308 of the arm may provide room for the physician to attend to the patient substantially free of interference from x-ray support structure 1318. As illustrated in FIG. 12B, the imaging system may include imaging device 1306 having a gantry housing 1324 attached to a support structure imaging device support structure 1328, such as a wheeled mobile cart 1330 with wheels 1332, which may enclose an image capturing portion, not illustrated. The image capturing portion may include an x-ray source and/or emission portion and an x-ray receiving and/or image receiving portion, which may be disposed about one hundred and eighty degrees from each other and mounted on a rotor (not illustrated) relative to a track of the image capturing portion. The image capturing portion may be operable to rotate three hundred and sixty degrees during image acquisition. The image capturing portion may rotate around a central point and/or axis, allowing image data of patient 210 to be acquired from multiple directions or in multiple planes. Although certain imaging systems 1304 are exemplified herein, it will be appreciated that any suitable imaging system may be selected by one of ordinary skill in the art.

Turning now to FIGS. 13A-13C, the medical robot system 100, 300, 600 relies on accurate positioning of the end-effector 112, 602, medical instruments 608, and/or the patient 210 (e.g., patient tracking device 116) relative to the desired medical area. In the embodiments shown in FIGS. 13A-13C, the tracking markers 118, 804 are rigidly attached to a portion of the instrument 608 and/or end-effector 112.

FIG. 13A depicts part of the medical robot system 100 with the robot 102 including base 106, robot arm 104, and end-effector 112. The other elements, not illustrated, such as the display, cameras, etc. may also be present as described herein. FIG. 13B depicts a close-up view of the end-effector 112 with guide tube 114 and a plurality of tracking markers 118 rigidly affixed to the end-effector 112. In this embodiment, the plurality of tracking markers 118 are attached to the guide tube 112. FIG. 13C depicts an instrument 608 (in this case, a probe 608A) with a plurality of tracking markers 804 rigidly affixed to the instrument 608. As described elsewhere herein, the instrument 608 could include any suitable medical instrument, such as, but not limited to, guide wire, cannula, a retractor, a drill, a reamer, a screw driver, an insertion tool, a removal tool, or the like.

When tracking an instrument 608, end-effector 112, or other object to be tracked in 3D, an array of tracking markers 118, 804 may be rigidly attached to a portion of the tool 608 or end-effector 112. Preferably, the tracking markers 118, 804 are attached such that the markers 118, 804 are out of the way (e.g., not impeding the medical operation, visibility, etc.). The markers 118, 804 may be affixed to the instrument 608, end-effector 112, or other object to be tracked, for example, with an array 612. Usually three or four markers 118, 804 are used with an array 612. The array 612 may include a linear section, a cross piece, and may be asymmetric such that the markers 118, 804 are at different relative positions and locations with respect to one another. For example, as shown in FIG. 13C, a probe 608A with a 4-marker tracking array 612 is shown, and FIG. 13B depicts the end-effector 112 with a different 4-marker tracking array 612.

In FIG. 13C, the tracking array 612 functions as the handle 620 of the probe 608A. Thus, the four markers 804 are attached to the handle 620 of the probe 608A, which is out of the way of the shaft 622 and tip 624. Stereophotogrammetric tracking of these four markers 804 allows the instrument 608 to be tracked as a rigid body and for the tracking system 100, 300, 600 to precisely determine the position of the tip 624 and the orientation of the shaft 622 while the probe 608A is moved around in front of tracking cameras 200, 326.

To enable automatic tracking of one or more tools 608, end-effector 112, or other object to be tracked in 3D (e.g., multiple rigid bodies), the markers 118, 804 on each tool 608, end-effector 112, or the like, are arranged asymmetrically with a known inter-marker spacing. The reason for asymmetric alignment is so that it is unambiguous which marker 118, 804 corresponds to a particular location on the rigid body and whether markers 118, 804 are being viewed from the front or back, i.e., mirrored. For example, if the markers 118, 804 were arranged in a square on the tool 608 or end-effector 112, it would be unclear to the system 100, 300, 600 which markers 118, 804 corresponded to which corner of the square. For example, for the probe 608A, it would be unclear which marker 804 was closest to the shaft 622. Thus, it would be unknown which way the shaft 622 was extending from the array 612. Accordingly, each array 612 and thus each tool 608, end-effector 112, or other object to be tracked should have a unique marker pattern to allow it to be distinguished from other tools 608 or other objects being tracked. Asymmetry and unique marker patterns allow the system 100, 300, 600 to detect individual markers 118, 804 then to check the marker spacing against a stored template to determine which tool 608, end effector 112, or other object they represent. Detected markers 118, 804 can then be sorted automatically and assigned to each tracked object in the correct order. Without this information, rigid body calculations could not then be performed to extract key geometric information, for example, such as tool tip 624 and alignment of the shaft 622, unless the user manually specified which detected marker 118, 804 corresponded to which position on each rigid body. These concepts are commonly known to those skilled in the methods of 3D optical tracking.

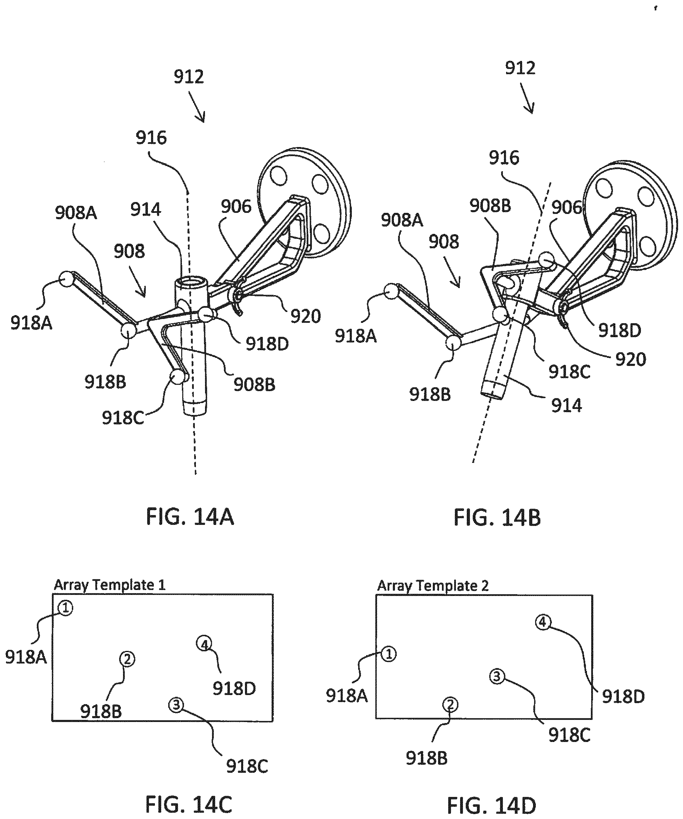

Turning now to FIGS. 14A-14D, an alternative version of an end-effector 912 with moveable tracking markers 918A-918D is shown. In FIG. 14A, an array with moveable tracking markers 918A-918D are shown in a first configuration, and in FIG. 14B the moveable tracking markers 918A-918D are shown in a second configuration, which is angled relative to the first configuration. FIG. 14C shows the template of the tracking markers 918A-918D, for example, as seen by the cameras 200, 326 in the first configuration of FIG. 14A; and FIG. 14D shows the template of tracking markers 918A-918D, for example, as seen by the cameras 200, 326 in the second configuration of FIG. 14B.

In this embodiment, 4-marker array tracking is contemplated wherein the markers 918A-918D are not all in fixed position relative to the rigid body and instead, one or more of the array markers 918A-918D can be adjusted, for example, during testing, to give updated information about the rigid body that is being tracked without disrupting the process for automatic detection and sorting of the tracked markers 918A-918D.

When tracking any tool, such as a guide tube 914 connected to the end effector 912 of a robot system 100, 300, 600, the tracking array's primary purpose is to update the position of the end effector 912 in the camera coordinate system. When using the rigid system, for example, as shown in FIG. 13B, the array 612 of reflective markers 118 rigidly extend from the guide tube 114. Because the tracking markers 118 are rigidly connected, knowledge of the marker locations in the camera coordinate system also provides exact location of the centerline, tip, and tail of the guide tube 114 in the camera coordinate system. Typically, information about the position of the end effector 112 from such an array 612 and information about the location of a target trajectory from another tracked source are used to calculate the required moves that must be input for each axis of the robot 102 that will move the guide tube 114 into alignment with the trajectory and move the tip to a particular location along the trajectory vector.

Sometimes, the desired trajectory is in an awkward or unreachable location, but if the guide tube 114 could be swiveled, it could be reached. For example, a very steep trajectory pointing away from the base 106 of the robot 102 might be reachable if the guide tube 114 could be swiveled upward beyond the limit of the pitch (wrist up-down angle) axis, but might not be reachable if the guide tube 114 is attached parallel to the plate connecting it to the end of the wrist. To reach such a trajectory, the base 106 of the robot 102 might be moved or a different end effector 112 with a different guide tube attachment might be exchanged with the working end effector. Both of these solutions may be time consuming and cumbersome.

As best seen in FIGS. 14A and 14B, if the array 908 is configured such that one or more of the markers 918A-918D are not in a fixed position and instead, one or more of the markers 918A-918D can be adjusted, swiveled, pivoted, or moved, the robot 102 can provide updated information about the object being tracked without disrupting the detection and tracking process. For example, one of the markers 918A-918D may be fixed in position and the other markers 918A-918D may be moveable; two of the markers 918A-918D may be fixed in position and the other markers 918A-918D may be moveable; three of the markers 918A-918D may be fixed in position and the other marker 918A-918D may be moveable; or all of the markers 918A-918D may be moveable.

In the embodiment shown in FIGS. 14A and 14B, markers 918A, 918 B are rigidly connected directly to a base 906 of the end-effector 912, and markers 918C, 918D are rigidly connected to the tube 914. Similar to array 612, array 908 may be provided to attach the markers 918A-918D to the end-effector 912, instrument 608, or other object to be tracked. In this case, however, the array 908 is comprised of a plurality of separate components. For example, markers 918A, 918B may be connected to the base 906 with a first array 908A, and markers 918C, 918D may be connected to the guide tube 914 with a second array 908B. Marker 918A may be affixed to a first end of the first array 908A and marker 918B may be separated a linear distance and affixed to a second end of the first array 908A. While first array 908 is substantially linear, second array 908B has a bent or V-shaped configuration, with respective root ends, connected to the guide tube 914, and diverging therefrom to distal ends in a V-shape with marker 918C at one distal end and marker 918D at the other distal end. Although specific configurations are exemplified herein, it will be appreciated that other asymmetric designs including different numbers and types of arrays 908A, 908B and different arrangements, numbers, and types of markers 918A-918D are contemplated.

The guide tube 914 may be moveable, swivelable, or pivotable relative to the base 906, for example, across a hinge 920 or other connector to the base 906. Thus, markers 918C, 918D are moveable such that when the guide tube 914 pivots, swivels, or moves, markers 918C, 918D also pivot, swivel, or move. As best seen in FIG. 14A, guide tube 914 has a longitudinal axis 916 which is aligned in a substantially normal or vertical orientation such that markers 918A-918D have a first configuration. Turning now to FIG. 14B, the guide tube 914 is pivoted, swiveled, or moved such that the longitudinal axis 916 is now angled relative to the vertical orientation such that markers 918A-918D have a second configuration, different from the first configuration.

In contrast to the embodiment described for FIGS. 14A-14D, if a swivel existed between the guide tube 914 and the arm 104 (e.g., the wrist attachment) with all four markers 918A-918D remaining attached rigidly to the guide tube 914 and this swivel was adjusted by the user, the robotic system 100, 300, 600 would not be able to automatically detect that the guide tube 914 orientation had changed. The robotic system 100, 300, 600 would track the positions of the marker array 908 and would calculate incorrect robot axis moves assuming the guide tube 914 was attached to the wrist (the robot arm 104) in the previous orientation. By keeping one or more markers 918A-918D (e.g., two markers 918C, 918D) rigidly on the tube 914 and one or more markers 918A-918D (e.g., two markers 918A, 918B) across the swivel, automatic detection of the new position becomes possible and correct robot moves are calculated based on the detection of a new tool or end-effector 112, 912 on the end of the robot arm 104.

One or more of the markers 918A-918D are configured to be moved, pivoted, swiveled, or the like according to any suitable means. For example, the markers 918A-918D may be moved by a hinge 920, such as a clamp, spring, lever, slide, toggle, or the like, or any other suitable mechanism for moving the markers 918A-918D individually or in combination, moving the arrays 908A, 908B individually or in combination, moving any portion of the end-effector 912 relative to another portion, or moving any portion of the tool 608 relative to another portion.

As shown in FIGS. 14A and 14B, the array 908 and guide tube 914 may become reconfigurable by simply loosening the clamp or hinge 920, moving part of the array 908A, 908B relative to the other part 908A, 908B, and retightening the hinge 920 such that the guide tube 914 is oriented in a different position. For example, two markers 918C, 918D may be rigidly interconnected with the tube 914 and two markers 918A, 918B may be rigidly interconnected across the hinge 920 to the base 906 of the end-effector 912 that attaches to the robot arm 104. The hinge 920 may be in the form of a clamp, such as a wing nut or the like, which can be loosened and retightened to allow the user to quickly switch between the first configuration (FIG. 14A) and the second configuration (FIG. 14B).

The cameras 200, 326 detect the markers 918A-918D, for example, in one of the templates identified in FIGS. 14C and 14D. If the array 908 is in the first configuration (FIG. 14A) and tracking cameras 200, 326 detect the markers 918A-918D, then the tracked markers match Array Template 1 as shown in FIG. 14C. If the array 908 is the second configuration (FIG. 14B) and tracking cameras 200, 326 detect the same markers 918A-918D, then the tracked markers match Array Template 2 as shown in FIG. 14D. Array Template 1 and Array Template 2 are recognized by the system 100, 300, 600 as two distinct tools, each with its own uniquely defined spatial relationship between guide tube 914, markers 918A-918D, and robot attachment. The user could therefore adjust the position of the end-effector 912 between the first and second configurations without notifying the system 100, 300, 600 of the change and the system 100, 300, 600 would appropriately adjust the movements of the robot 102 to stay on trajectory.

In this embodiment, there are two assembly positions in which the marker array matches unique templates that allow the system 100, 300, 600 to recognize the assembly as two different tools or two different end effectors. In any position of the swivel between or outside of these two positions (namely, Array Template 1 and Array Template 2 shown in FIGS. 14C and 14D, respectively), the markers 918A-918D would not match any template and the system 100, 300, 600 would not detect any array present despite individual markers 918A-918D being detected by cameras 200, 326, with the result being the same as if the markers 918A-918D were temporarily blocked from view of the cameras 200, 326. It will be appreciated that other array templates may exist for other configurations, for example, identifying different instruments 608 or other end-effectors 112, 912, etc.

In the embodiment described, two discrete assembly positions are shown in FIGS. 14A and 14B. It will be appreciated, however, that there could be multiple discrete positions on a swivel joint, linear joint, combination of swivel and linear joints, pegboard, or other assembly where unique marker templates may be created by adjusting the position of one or more markers 918A-918D of the array relative to the others, with each discrete position matching a particular template and defining a unique tool 608 or end-effector 112, 912 with different known attributes. In addition, although exemplified for end effector 912, it will be appreciated that moveable and fixed markers 918A-918D may be used with any suitable instrument 608 or other object to be tracked.

When using an external 3D tracking system 100, 300, 600 to track a full rigid body array of three or more markers attached to a robot's end effector 112 (for example, as depicted in FIGS. 13A and 13B), it is possible to directly track or to calculate the 3D position of every section of the robot 102 in the coordinate system of the cameras 200, 326. The geometric orientations of joints relative to the tracker are known by design, and the linear or angular positions of joints are known from encoders for each motor of the robot 102, fully defining the 3D positions of all of the moving parts from the end effector 112 to the base 116. Similarly, if a tracker were mounted on the base 106 of the robot 102 (not shown), it is likewise possible to track or calculate the 3D position of every section of the robot 102 from base 106 to end effector 112 based on known joint geometry and joint positions from each motor's encoder.

In some situations, it may be desirable to track the positions of all segments of the robot 102 from fewer than three markers 118 rigidly attached to the end effector 112. Specifically, if a tool 608 is introduced into the guide tube 114, it may be desirable to track full rigid body motion of the robot 902 with only one additional marker 118 being tracked.

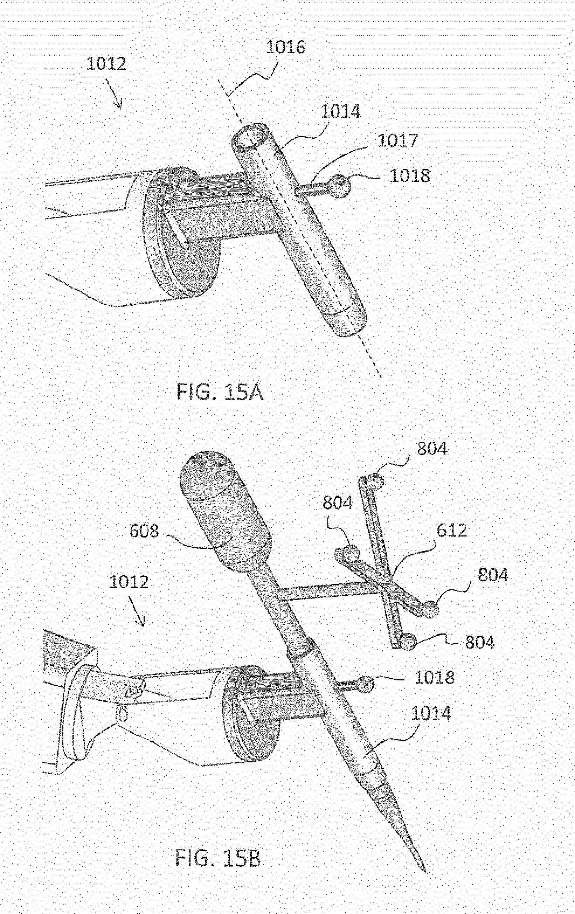

Turning now to FIGS. 15A-15E, an alternative version of an end-effector 1012 having only a single tracking marker 1018 is shown. End-effector 1012 may be similar to the other end-effectors described herein, and may include a guide tube 1014 extending along a longitudinal axis 1016. A single tracking marker 1018, similar to the other tracking markers described herein, may be rigidly affixed to the guide tube 1014. This single marker 1018 can serve the purpose of adding missing degrees of freedom to allow full rigid body tracking and/or can serve the purpose of acting as a surveillance marker to ensure that assumptions about robot and camera positioning are valid.

The single tracking marker 1018 may be attached to the robotic end effector 1012 as a rigid extension to the end effector 1012 that protrudes in any convenient direction and does not obstruct the doctor's view. The tracking marker 1018 may be affixed to the guide tube 1014 or any other suitable location of on the end-effector 1012. When affixed to the guide tube 1014, the tracking marker 1018 may be positioned at a location between first and second ends of the guide tube 1014. For example, in FIG. 15A, the single tracking marker 1018 is shown as a reflective sphere mounted on the end of a narrow shaft 1017 that extends forward from the guide tube 1014 and is positioned longitudinally above a mid-point of the guide tube 1014 and below the entry of the guide tube 1014. This position allows the marker 1018 to be generally visible by cameras 200, 326 but also would not obstruct vision of the doctor 120 or collide with other tools or objects in the vicinity of surgery. In addition, the guide tube 1014 with the marker 1018 in this position is designed for the marker array on any tool 608 introduced into the guide tube 1014 to be visible at the same time as the single marker 1018 on the guide tube 1014 is visible.

As shown in FIG. 15B, when a snugly fitting tool or instrument 608 is placed within the guide tube 1014, the instrument 608 becomes mechanically constrained in 4 of 6 degrees of freedom. That is, the instrument 608 cannot be rotated in any direction except about the longitudinal axis 1016 of the guide tube 1014 and the instrument 608 cannot be translated in any direction except along the longitudinal axis 1016 of the guide tube 1014. In other words, the instrument 608 can only be translated along and rotated about the centerline of the guide tube 1014. If two more parameters are known, such as (1) an angle of rotation about the longitudinal axis 1016 of the guide tube 1014; and (2) a position along the guide tube 1014, then the position of the end effector 1012 in the camera coordinate system becomes fully defined.

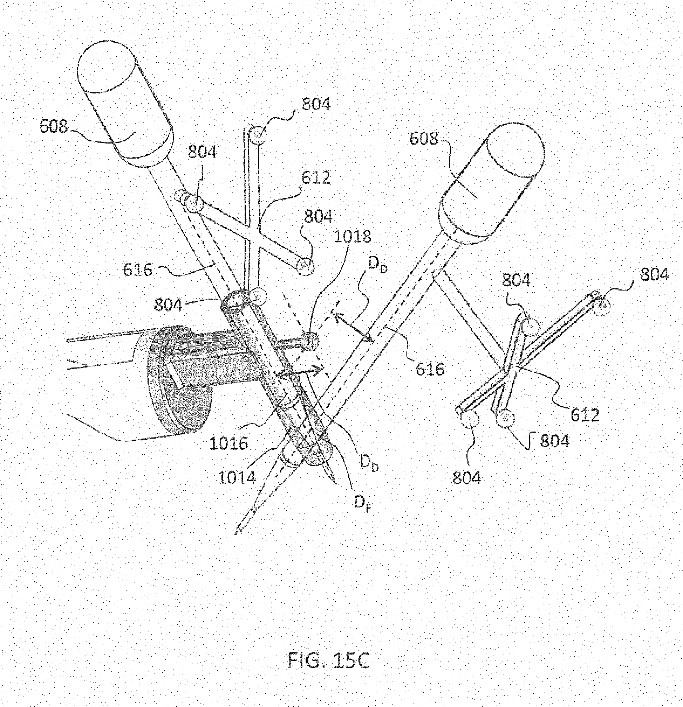

Referring now to FIG. 15C, the system 100, 300, 600 should be able to know when a tool 608 is actually positioned inside of the guide tube 1014 and is not instead outside of the guide tube 1014 and just somewhere in view of the cameras 200, 326. The tool 608 has a longitudinal axis or centerline 616 and an array 612 with a plurality of tracked markers 804. The rigid body calculations may be used to determine where the centerline 616 of the tool 608 is located in the camera coordinate system based on the tracked position of the array 612 on the tool 608.

The fixed normal (perpendicular) distance D_{F} from the single marker 1018 to the centerline or longitudinal axis 1016 of the guide tube 1014 is fixed and is known geometrically, and the position of the single marker 1018 can be tracked. Therefore, when a detected distance D_{D} from tool centerline 616 to single marker 1018 matches the known fixed distance D_{F} from the guide tube centerline 1016 to the single marker 1018, it can be determined that the tool 608 is either within the guide tube 1014 (centerlines 616, 1016 of tool 608 and guide tube 1014 coincident) or happens to be at some point in the locus of possible positions where this distance D_{D} matches the fixed distance D_{F}. For example, in FIG. 15C, the normal detected distance D_{D} from tool centerline 616 to the single marker 1018 matches the fixed distance D_{F} from guide tube centerline 1016 to the single marker 1018 in both frames of data (tracked marker coordinates) represented by the transparent tool 608 in two positions, and thus, additional considerations may be needed to determine when the tool 608 is located in the guide tube 1014.

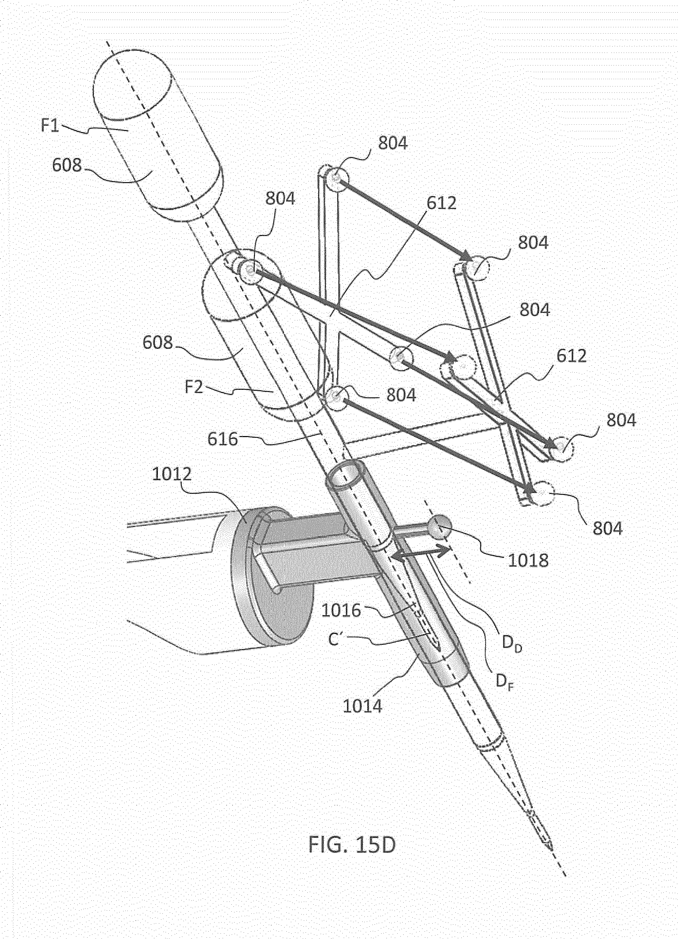

Turning now to FIG. 15D, programmed logic can be used to look for frames of tracking data in which the detected distance D_{D} from tool centerline 616 to single marker 1018 remains fixed at the correct length despite the tool 608 moving in space by more than some minimum distance relative to the single sphere 1018 to satisfy the condition that the tool 608 is moving within the guide tube 1014. For example, a first frame F1 may be detected with the tool 608 in a first position and a second frame F2 may be detected with the tool 608 in a second position (namely, moved linearly with respect to the first position). The markers 804 on the tool array 612 may move by more than a given amount (e.g., more than 5mm total) from the first frame F1 to the second frame F2. Even with this movement, the detected distance D_{D} from the tool centerline vector C' to the single marker 1018 is substantially identical in both the first frame F1 and the second frame F2.

Logistically, the doctor 120 or user could place the tool 608 within the guide tube 1014 and slightly rotate it or slide it down into the guide tube 1014 and the system 100, 300, 600 would be able to detect that the tool 608 is within the guide tube 1014 from tracking of the five markers (four markers 804 on tool 608 plus single marker 1018 on guide tube 1014). Knowing that the tool 608 is within the guide tube 1014, all 6 degrees of freedom may be calculated that define the position and orientation of the robotic end effector 1012 in space. Without the single marker 1018, even if it is known with certainty that the tool 608 is within the guide tube 1014, it is unknown where the guide tube 1014 is located along the tool's centerline vector C' and how the guide tube 1014 is rotated relative to the centerline vector C'.

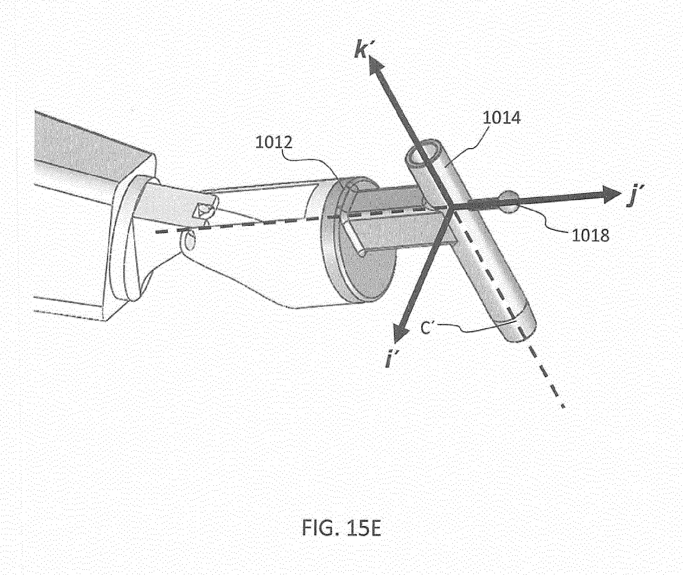

With emphasis on FIG. 15E, the presence of the single marker 1018 being tracked as well as the four markers 804 on the tool 608, it is possible to construct the centerline vector C' of the guide tube 1014 and tool 608 and the normal vector through the single marker 1018 and through the centerline vector C'. This normal vector has an orientation that is in a known orientation relative to the forearm of the robot distal to the wrist (in this example, oriented parallel to that segment) and intersects the centerline vector C' at a specific fixed position. For convenience, three mutually orthogonal vectors k', j', i' can be constructed, as shown in FIG. 15E, defining rigid body position and orientation of the guide tube 1014. One of the three mutually orthogonal vectors k' is constructed from the centerline vector C', the second vector j' is constructed from the normal vector through the single marker 1018, and the third vector i' is the vector cross product of the first and second vectors k', j'. The robot's joint positions relative to these vectors k', j', i' are known and fixed when all joints are at zero, and therefore rigid body calculations can be used to determine the location of any section of the robot relative to these vectors k', j', i' when the robot is at a home position. During robot movement, if the positions of the tool markers 804 (while the tool 608 is in the guide tube 1014) and the position of the single marker 1018 are detected from the tracking system, and angles/linear positions of each joint are known from encoders, then position and orientation of any section of the robot can be determined.

In some embodiments, it may be useful to fix the orientation of the tool 608 relative to the guide tube 1014. For example, the end effector guide tube 1014 may be oriented in a particular position about its axis 1016 to allow machining or implant positioning. Although the orientation of anything attached to the tool 608 inserted into the guide tube 1014 is known from the tracked markers 804 on the tool 608, the rotational orientation of the guide tube 1014 itself in the camera coordinate system is unknown without the additional tracking marker 1018 (or multiple tracking markers in other embodiments) on the guide tube 1014. This marker 1018 provides essentially a "clock position" from -180° to +180° based on the orientation of the marker 1018 relative to the centerline vector C'. Thus, the single marker 1018 can provide additional degrees of freedom to allow full rigid body tracking and/or can act as a surveillance marker to ensure that assumptions about the robot and camera positioning are valid.

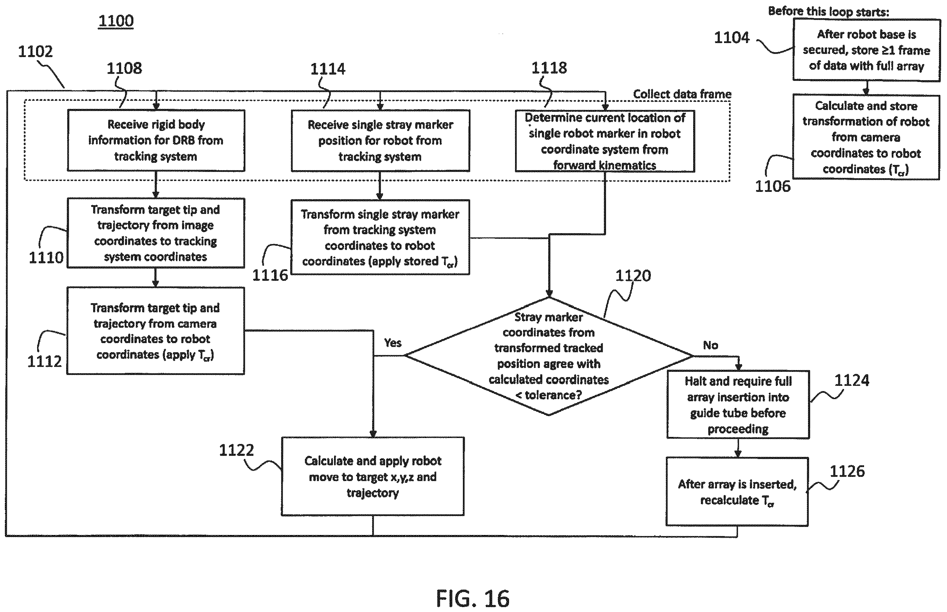

FIG. 16 is a block diagram of a method 1100 for navigating and moving the end-effector 1012 (or any other end-effector described herein) of the robot 102 to a desired target trajectory. Another use of the single marker 1018 on the robotic end effector 1012 or guide tube 1014 is as part of the method 1100 enabling the automated safe movement of the robot 102 without a full tracking array attached to the robot 102. This method 1100 functions when the tracking cameras 200, 326 do not move relative to the robot 102 (i.e., they are in a fixed position), the tracking system's coordinate system and robot's coordinate system are co-registered, and the robot 102 is calibrated such that the position and orientation of the guide tube 1014 can be accurately determined in the robot's Cartesian coordinate system based only on the encoded positions of each robotic axis.

For this method 1100, the coordinate systems of the tracker and the robot must be co-registered, meaning that the coordinate transformation from the tracking system's Cartesian coordinate system to the robot's Cartesian coordinate system is needed. For convenience, this coordinate transformation can be a 4x4 matrix of translations and rotations that is well known in the field of robotics. This transformation will be termed Tcr to refer to "transformation - camera to robot". Once this transformation is known, any new frame of tracking data, which is received as x,y,z coordinates in vector form for each tracked marker, can be multiplied by the 4x4 matrix and the resulting x,y,z coordinates will be in the robot's coordinate system. To obtain Tcr, a full tracking array on the robot is tracked while it is rigidly attached to the robot at a location that is known in the robot's coordinate system, then known rigid body methods are used to calculate the transformation of coordinates. It should be evident that any tool 608 inserted into the guide tube 1014 of the robot 102 can provide the same rigid body information as a rigidly attached array when the additional marker 1018 is also read. That is, the tool 608 need only be inserted to any position within the guide tube 1014 and at any rotation within the guide tube 1014, not to a fixed position and orientation. Thus, it is possible to determine Tcr by inserting any tool 608 with a tracking array 612 into the guide tube 1014 and reading the tool's array 612 plus the single marker 1018 of the guide tube 1014 while at the same time determining from the encoders on each axis the current location of the guide tube 1014 in the robot's coordinate system.

Logic for navigating and moving the robot 102 to a target trajectory is provided in the method 1100 of FIG. 16. Before entering the loop 1102, it is assumed that the transformation Tcr was previously stored. Thus, before entering loop 1102, in step 1104, after the robot base 106 is secured, greater than or equal to one frame of tracking data of a tool inserted in the guide tube while the robot is static is stored; and in step 1106, the transformation of robot guide tube position from camera coordinates to robot coordinates Tcr is calculated from this static data and previous calibration data. Tcr should remain valid as long as the cameras 200, 326 do not move relative to the robot 102. If the cameras 200, 326 move relative to the robot 102, and Tcr needs to be re-obtained, the system 100, 300, 600 can be made to prompt the user to insert a tool 608 into the guide tube 1014 and then automatically perform the necessary calculations.

In the flowchart of method 1100, each frame of data collected consists of the tracked position of the DRB 1404 on the patient 210, the tracked position of the single marker 1018 on the end effector 1014, and a snapshot of the positions of each robotic axis. From the positions of the robot's axes, the location of the single marker 1018 on the end effector 1012 is calculated. This calculated position is compared to the actual position of the marker 1018 as recorded from the tracking system. If the values agree, it can be assured that the robot 102 is in a known location. The transformation Tcr is applied to the tracked position of the DRB 1404 so that the target for the robot 102 can be provided in terms of the robot's coordinate system. The robot 102 can then be commanded to move to reach the target.

After steps 1104, 1106, loop 1102 includes step 1108 receiving rigid body information for DRB 1404 from the tracking system; step 1110 transforming target tip and trajectory from image coordinates to tracking system coordinates; and step 1112 transforming target tip and trajectory from camera coordinates to robot coordinates (apply Tcr). Loop 1102 further includes step 1114 receiving a single stray marker position for robot from tracking system; and step 1116 transforming the single stray marker from tracking system coordinates to robot coordinates (apply stored Tcr). Loop 1102 also includes step 1118 determining current location of the single robot marker 1018 in the robot coordinate system from forward kinematics. The information from steps 1116 and 1118 is used to determine step 1120 whether the stray marker coordinates from transformed tracked position agree with the calculated coordinates being less than a given tolerance. If yes, proceed to step 1122, calculate and apply robot move to target x, y, z and trajectory. If no, proceed to step 1124, halt and require full array insertion into guide tube 1014 before proceeding; step 1126 after array is inserted, recalculate Tcr; and then proceed to repeat steps 1108, 1114, and 1118.

This method 1100 has advantages over a method in which the continuous monitoring of the single marker 1018 to verify the location is omitted. Without the single marker 1018, it would still be possible to determine the position of the end effector 1012 using Tcr and to send the end-effector 1012 to a target location but it would not be possible to verify that the robot 102 was actually in the expected location. For example, if the cameras 200, 326 had been bumped and Tcr was no longer valid, the robot 102 would move to an erroneous location. For this reason, the single marker 1018 provides value with regard to safety.

For a given fixed position of the robot 102, it is theoretically possible to move the tracking cameras 200, 326 to a new location in which the single tracked marker 1018 remains unmoved since it is a single point, not an array. In such a case, the system 100, 300, 600 would not detect any error since there would be agreement in the calculated and tracked locations of the single marker 1018. However, once the robot's axes caused the guide tube 1012 to move to a new location, the calculated and tracked positions would disagree and the safety check would be effective.

The term "surveillance marker" may be used, for example, in reference to a single marker that is in a fixed location relative to the DRB 1404. In this instance, if the DRB 1404 is bumped or otherwise dislodged, the relative location of the surveillance marker changes and the doctor 120 can be alerted that there may be a problem with navigation. Similarly, in the embodiments described herein, with a single marker 1018 on the robot's guide tube 1014, the system 100, 300, 600 can continuously check whether the cameras 200, 326 have moved relative to the robot 102. If registration of the tracking system's coordinate system to the robot's coordinate system is lost, such as by cameras 200, 326 being bumped or malfunctioning or by the robot malfunctioning, the system 100, 300, 600 can alert the user and corrections can be made. Thus, this single marker 1018 can also be thought of as a surveillance marker for the robot 102.

It should be clear that with a full array permanently mounted on the robot 102 (e.g., the plurality of tracking markers 702 on end-effector 602 shown in FIGS. 7A-7C) such functionality of a single marker 1018 as a robot surveillance marker is not needed because it is not required that the cameras 200, 326 be in a fixed position relative to the robot 102, and Tcr is updated at each frame based on the tracked position of the robot 102. Reasons to use a single marker 1018 instead of a full array are that the full array is more bulky and obtrusive, thereby blocking the doctor's view and access to the medical field 208 more than a single marker 1018, and line of sight to a full array is more easily blocked than line of sight to a single marker 1018.

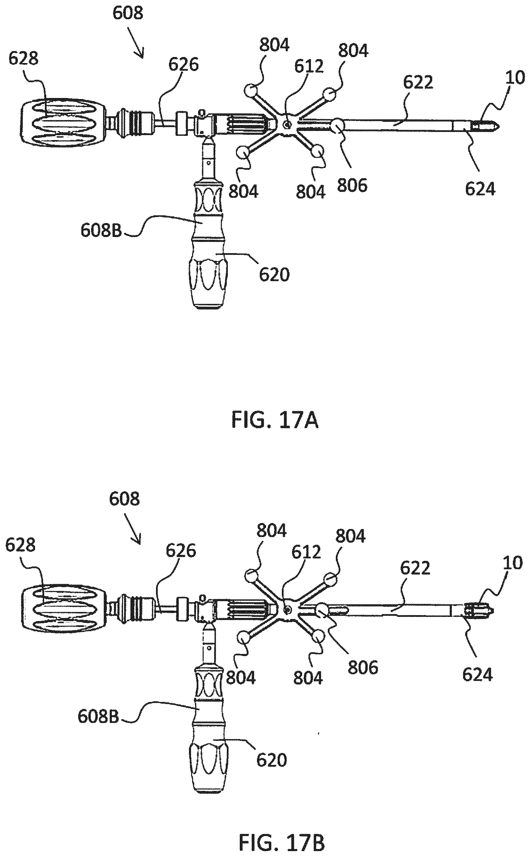

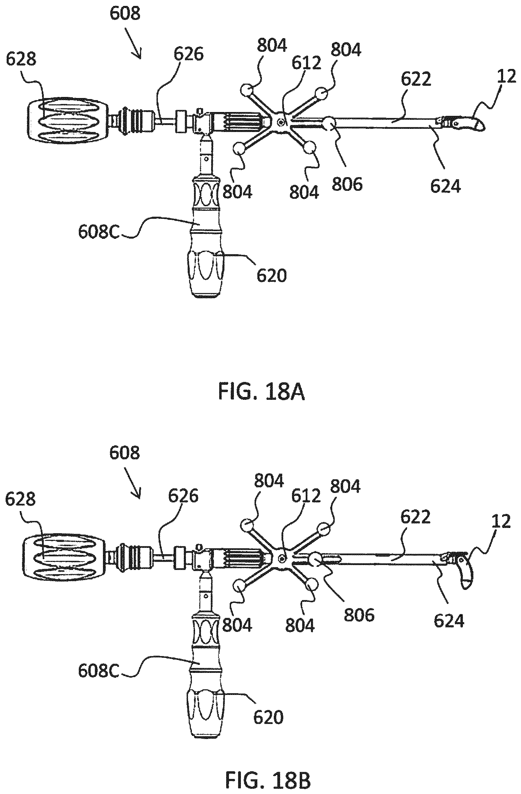

Turning now to FIGS. 17A-17B and 18A-18B, instruments 608, such as implant holders 608B, 608C, are depicted which include both fixed and moveable tracking markers 804, 806. The implant holders 608B, 608C may have a handle 620 and an outer shaft 622 extending from the handle 620. The shaft 622 may be positioned substantially perpendicular to the handle 620, as shown, or in any other suitable orientation. An inner shaft 626 may extend through the outer shaft 622 with a knob 628 at one end. Implant 10, 12 connects to the shaft 622, at the other end, at tip 624 of the implant holder 608B, 608C using typical connection mechanisms known to those of skill in the art. The knob 628 may be rotated, for example, to expand or articulate the implant 10, 12. U.S. Patent Nos. 8,709,086 and 8,491,659, which are incorporated by reference herein, describe expandable fusion devices and methods of installation.

When tracking the tool 608, such as implant holder 608B, 608C, the tracking array 612 may contain a combination of fixed markers 804 and one or more moveable markers 806 which make up the array 612 or is otherwise attached to the implant holder 608B, 608C. The navigation array 612 may include at least one or more (e.g., at least two) fixed position markers 804, which are positioned with a known location relative to the implant holder instrument 608B, 608C. These fixed markers 804 would not be able to move in any orientation relative to the instrument geometry and would be useful in defining where the instrument 608 is in space. In addition, at least one marker 806 is present which can be attached to the array 612 or the instrument itself which is capable of moving within a pre-determined boundary (e.g., sliding, rotating, etc.) relative to the fixed markers 804. The system 100, 300, 600 (e.g., the software) correlates the position of the moveable marker 806 to a particular position, orientation, or other attribute of the implant 10 (such as height of an expandable interbody spacer shown in FIGS. 17A-17B or angle of an articulating interbody spacer shown in FIGS. 18A-18B). Thus, the system and/or the user can determine the height or angle of the implant 10, 12 based on the location of the moveable marker 806.

In the embodiment shown in FIGS. 17A-17B, four fixed markers 804 are used to define the implant holder 608B and a fifth moveable marker 806 is able to slide within a pre-determined path to provide feedback on the implant height (e.g., a contracted position or an expanded position). FIG. 17A shows the expandable spacer 10 at its initial height, and FIG. 17B shows the spacer 10 in the expanded state with the moveable marker 806 translated to a different position. In this case, the moveable marker 806 moves closer to the fixed markers 804 when the implant 10 is expanded, although it is contemplated that this movement may be reversed or otherwise different. The amount of linear translation of the marker 806 would correspond to the height of the implant 10. Although only two positions are shown, it would be possible to have this as a continuous function whereby any given expansion height could be correlated to a specific position of the moveable marker 806.

Turning now to FIGS. 18A-18B, four fixed markers 804 are used to define the implant holder 608C and a fifth, moveable marker 806 is configured to slide within a pre-determined path to provide feedback on the implant articulation angle. FIG. 18A shows the articulating spacer 12 at its initial linear state, and FIG. 18B shows the spacer 12 in an articulated state at some offset angle with the moveable marker 806 translated to a different position. The amount of linear translation of the marker 806 would correspond to the articulation angle of the implant 12. Although only two positions are shown, it would be possible to have this as a continuous function whereby any given articulation angle could be correlated to a specific position of the moveable marker 806.

In these embodiments, the moveable marker 806 slides continuously to provide feedback about an attribute of the implant 10, 12 based on position. It is also contemplated that there may be discreet positions that the moveable marker 806 must be in which would also be able to provide further information about an implant attribute. In this case, each discreet configuration of all markers 804, 806 correlates to a specific geometry of the implant holder 608B, 608C and the implant 10, 12 in a specific orientation or at a specific height. In addition, any motion of the moveable marker 806 could be used for other variable attributes of any other type of navigated implant.

Although depicted and described with respect to linear movement of the moveable marker 806, the moveable marker 806 should not be limited to just sliding as there may be applications where rotation of the marker 806 or other movements could be useful to provide information about the implant 10, 12. Any relative change in position between the set of fixed markers 804 and the moveable marker 806 could be relevant information for the implant 10, 12 or other device. In addition, although expandable and articulating implants 10, 12 are exemplified, the instrument 608 could work with other medical devices and materials, such as spacers, cages, plates, fasteners, nails, screws, rods, pins, wire structures, sutures, anchor clips, staples, stents, bone grafts, biologics, cements, or the like.

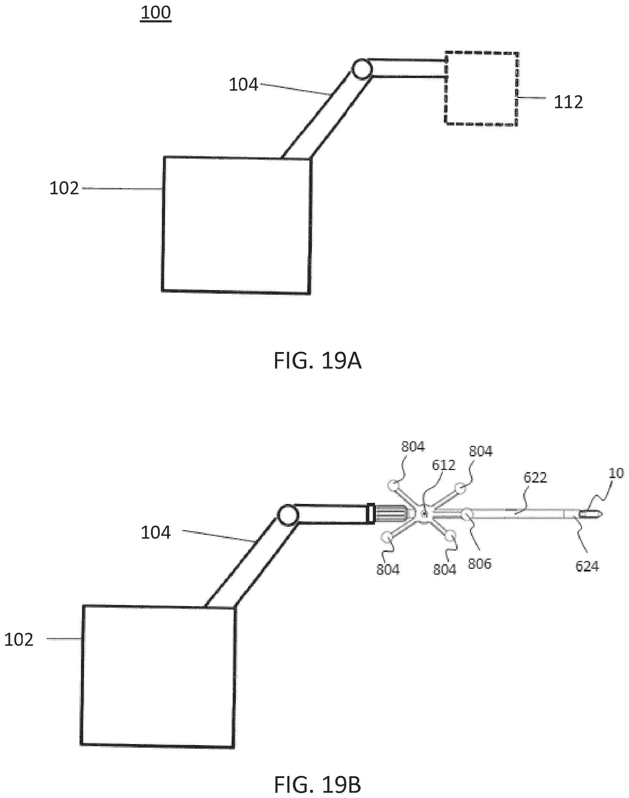

Turning now to FIG. 19A, it is envisioned that the robot end-effector 112 is interchangeable with other types of end-effectors 112. Moreover, it is contemplated that each end-effector 112 may be able to perform one or more functions based on a desired medical procedure. For example, the end-effector 112 having a guide tube 114 may be used for guiding an instrument 608 as described herein. In addition, end-effector 112 may be replaced with a different or alternative end-effector 112 that controls a medical device, instrument, or implant, for example.

The alternative end-effector 112 may include one or more devices or instruments coupled to and controllable by the robot. By way of non-limiting example, the end-effector 112, as depicted in FIG. 19A, may comprise a retractor (for example, one or more retractors disclosed in U.S. Patent Nos. 8,992,425 and 8,968,363) or one or more mechanisms for inserting or installing medical devices such as expandable intervertebral fusion devices (such as expandable implants exemplified in U.S. Patent Nos. 8,845,734; 9,510,954; and 9,456,903), stand-alone intervertebral fusion devices (such as implants exemplified in U.S. Patent Nos. 9,364,343 and 9,480,579), expandable corpectomy devices (such as corpectomy implants exemplified in U.S. Patent Nos. 9,393,128 and 9,173,747), articulating spacers (such as implants exemplified in U.S. Patent No. 9,259,327), facet prostheses (such as devices exemplified in U.S. Patent No. 9,539,031), laminoplasty devices (such as devices exemplified in U.S. Patent No. 9,486,253), spinous process spacers (such as implants exemplified in U.S. Patent No. 9,592,082), inflatables, fasteners including polyaxial screws, uniplanar screws, pedicle screws, posted screws, and the like, bone fixation plates, rod constructs and revision devices (such as devices exemplified in U.S. Patent No. 8,882,803), artificial and natural discs, motion preserving devices and implants, spinal cord stimulators (such as devices exemplified in U.S. Patent No. 9,440,076), and other medical devices. The end-effector 112 may include one or instruments directly or indirectly coupled to the robot for providing bone cement, bone grafts, living cells, pharmaceuticals, or other deliverable to a medical target. The end-effector 112 may also include one or more instruments designed for performing a discectomy, kyphoplasty, vertebrostenting, dilation, or other medical procedure.

The end-effector itself and/or the implant, device, or instrument may include one or more markers 118 such that the location and position of the markers 118 may be identified in three-dimensions. It is contemplated that the markers 118 may include active or passive markers 118, as described herein, that may be directly or indirectly visible to the cameras 200. Thus, one or more markers 118 located on an implant 10, for example, may provide for tracking of the implant 10 before, during, and after implantation.

As shown in FIG. 19B, the end-effector 112 may include an instrument 608 or portion thereof that is coupled to the robot arm 104 (for example, the instrument 608 may be coupled to the robot arm 104 by the coupling mechanism shown in FIGS. 9A-9C) and is controllable by the robot system 100. Thus, in the embodiment shown in FIG. 19B, the robot system 100 is able to insert implant 10 into a patient and expand or contract the expandable implant 10. Accordingly, the robot system 100 may be configured to assist a doctor or to operate partially or completely independently thereof. Thus, it is envisioned that the robot system 100 may be capable of controlling each alternative end-effector 112 for its specified function or medical procedure.

Although the robot and associated systems described herein are generally described with reference to spine applications, it is also contemplated that the robot system may be configured for use in other medical applications, including but not limited to, medical imaging, surgeries in trauma or other orthopedic applications (such as the placement of intramedullary nails, plates, and the like), cranial, neuro, cardiothoracic, vascular, colorectal, oncological, dental, and other medical operations and procedures.

During robotic spine (or other) procedures, a Dynamic Reference Base (DRB) may thus be affixed to the patient (e.g., to a bone of the patient), and used to track the patient anatomy. Since the patient is breathing, a position of the DRB (which is attached to the patient's body) may oscillate. Once a medical tool is robotically moved to a target trajectory and locked into position, patient movement (e.g., due to breathing) may cause deviation from the target trajectory even through the end-effector (e.g., medical tool) is locked in place. This deviation/shift (if unnoticed and unaccounted for) may thus reduce accuracy of the system and/or medical procedure.

Elements of robotic systems discussed above may be used to adjust placement of a virtual implant according to some embodiments of inventive concepts. By using navigated tools to plan instrument and implant trajectories, surgeons can have a more user-friendly system and more flexibility when planning robotic or navigated surgical cases. Setting the offset along a planned trajectory may allow individuals familiar with surgical navigation to pick up robotic surgery more quickly, and allow more flexibility for all users in planning interventions. The system can offer surgeons greater flexibility because the motions performed in moving the probe to position the virtual implant may mimic the motions of a surgery to be performed to implant a physical implant in a body. The system can also make planning implants with correct offsets easier and better leverage the perspective of the surgeon.

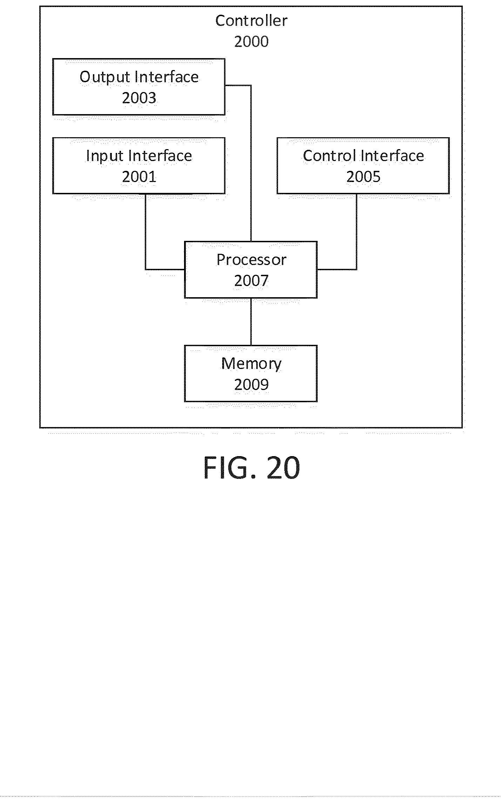

FIG. 20 is a block diagram illustrating elements of a controller 2000 (e.g., implemented within computer 408) for a robotic navigation system (also referred to as an imaging system or a surgical navigation system). As shown, controller 2000 may include processor circuit 2007 (also referred to as a processor) coupled with input interface circuit 2001 (also referred to as an input interface), output interface circuit 2003 (also referred to as an output interface), control interface circuit 2005 (also referred to as a control interface, and memory circuit 2009 (also referred to as a memory). The memory circuit 2009 may include computer readable program code that when executed by the processor circuit 2007 causes the processor circuit to perform operations according to embodiments disclosed herein. According to other embodiments, processor circuit 2007 may be defined to include memory so that a separate memory circuit is not required.

As discussed herein, operations of navigation system controller 2000 (e.g., a robotic navigation system controller) may be performed by processor 2007, input interface 2001, output interface 2003, and/or control interface 2005. For example, processor 2007 may receive user input through input interface 2001, and such user input may include user input received through a keyboard, mouse, touch sensitive display, foot pedal 544, tablet 546, etc. Processor 2007 may also receive position sensor input from tracking system 532 and/or cameras 200 through input interface 2001. Processor 2007 may provide output through output interface 2003, and such output may include information to render graphic/visual information on display 304 and/or audio output to be provided through speaker 536. Processor 2007 may provide robotic control information through control interface 2005 to motion control subsystem 506, and the robotic control information may be used to control operation of robot arm 104 or other robotic actuators. Processor 2007 may also receive feedback information through control interface 2005.

FIG. 21 depicts an embodiment according to the invention of a surgical navigation system used to adjust placement of a virtual implant 2120 in response to movement of a probe 2110. In this embodiment of the invention, the surgical navigation system includes sensors 2160, which can capture movement of a probe 2110 with a tip 2112 in relation to a body 2150. The sensors 2160 can include cameras 200, the probe 2110 can include navigated instrument 608, and the body 2150 can include patient 210. The sensors 2160 detect tracking markers 2111 attached to the probe 2110 and determine positional data for the probe 2110 based on the position of the tracking markers 2111. The surgical navigation system determines a trajectory 2140 based on an orientation of the probe 2110 and determines a position of a virtual implant 2120 based on an offset 2130 from the tip 2112 along the trajectory 2140.

According to the invention, the probe 2110 is a physical device that is positioned adjacent to the body 2150, which can include a human body. The surgical navigation system uses 3D anatomical image information to generate an image of the body 2150 that includes the virtual implant 2120. The image including the virtual implant 2120 is displayed on a monitor and updated such that moving the probe 2110 adjacent to the body 2150 causes a virtual implementation of the probe 2110, trajectory 2140, or the virtual implant 2120 to move on the image displayed. The system tracks the probe 2110, and on a first press of a button (e.g., a foot pedal) set the trajectory 2112 and determine a placement for the virtual implant 2120 at a default offset along the trajectory 2140. Holding the button can allow for further adjustment of the offset 2130 along the trajectory 2140. Adjustments to the offset 2130 can be calculated in several ways.

In some embodiments, the position of the tip 2112 of the probe 2110 can be used to determine the position of the virtual implant 2120 along the trajectory 2140. In additional or alternative embodiments, the position of the virtual implant 2120 can be adjusted based on detecting a rotation of the probe 2110.

For purposes of illustration, Figure 21 mixes physical elements (directly visible to a user) and virtual elements (that are only visible on a display). In particular, a surface of body 2150 and/or the physical probe 2110 may be visible to the user and sensor 2160, but internal portions of the body and the virtual implant are not directly visible. Instead, the surgical navigation system may render a two dimensional image on a display, with the two dimensional image including internal portions of body 2150, virtual implant 2120, the surface of body 2150, and probe 2110.

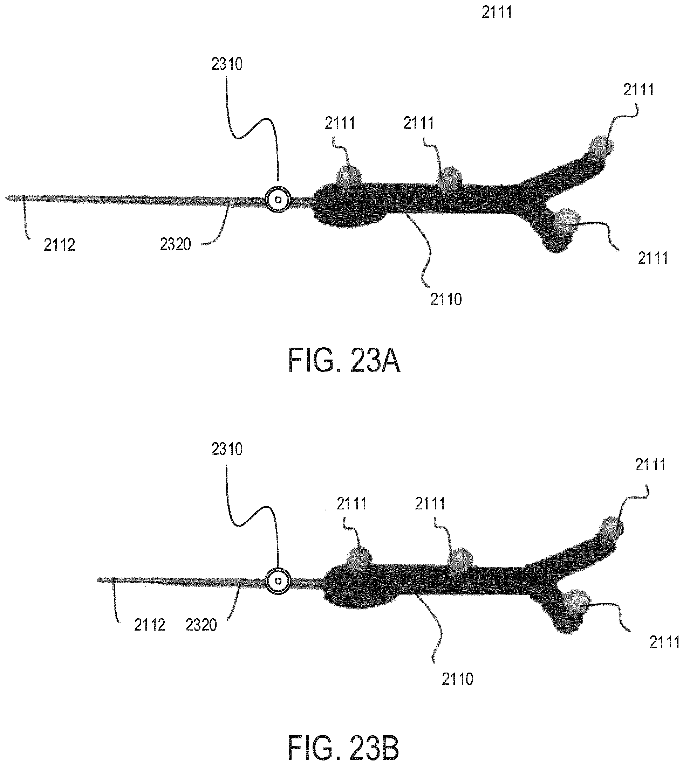

Examples of a telescoping version of the probe 2110 are depicted in FIGS. 23A-B. The same probe 2110 is depicted in both FIG. 23A and 23B. The probe includes a thumb wheel 2310 that may be turned to adjust a length of the probe 2110. By turning the thumb wheel 2310 in FIG. 23A clockwise the telescoping shaft 2320 retracts upon itself and shortens to the length of the telescoping shaft 2320 in FIG. 23B. In some embodiments, processor 2007 can be provided with positional data for the tracking markers 2111 and a default length of the tip 2112 from the tracking markers 2111. The processor 2007 can determine the position of the virtual implant 2120 based on the positional data for the tracking markers 2111 and the default length such that if a user adjusts the actual length of the probe 2110 while maintaining the position of the tip 2112 processor 2007 can adjust the position of the virtual implant. For example, if a user shortens a probe 2110 using the thumb wheel 2310 while maintaining the position of the tip 2112, the processor 2007 will calculate the position of the tip 2112 to be closer (or farther within) the anatomical body 2150 than it is and cause the position of the virtual implant 2120 to be adjusted to be farther into the body 2150.

Although FIGS. 23A-B depict a telescoping version of the probe 2110 with a thumb wheel 2310, other mechanisms can be used to adjust the length of the probe 2110. The probe 2110 is explained with more detail in regards to the medical instrument in FIGS. 13A-C above.

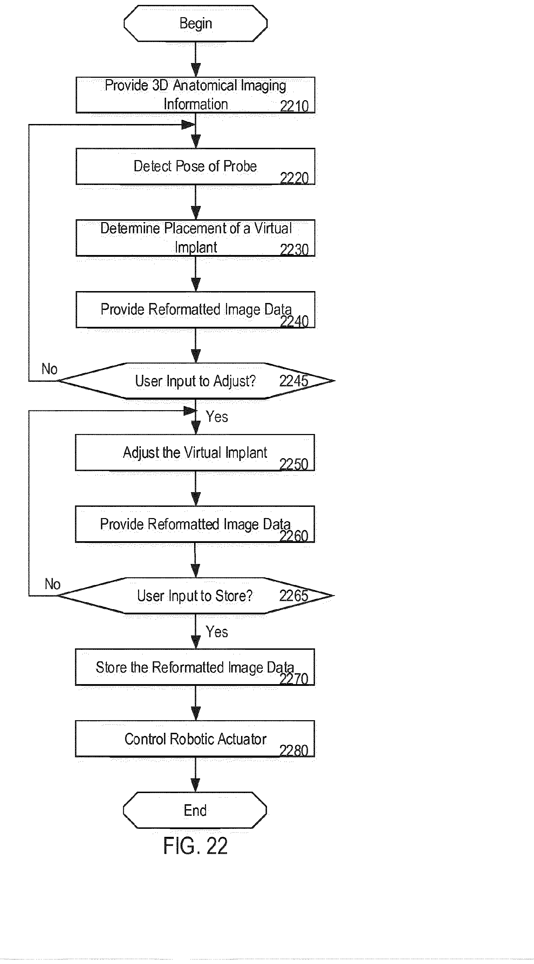

Operations of a navigation system (e.g., a robotic navigation system that includes a robotic actuator configured to position a probe on a body) will now be discussed with reference to the flow chart of FIG. 22 according to some embodiments of inventive concepts. For example, modules may be stored in memory 2009 of FIG. 20, and these modules may provide instructions so that when the instructions of a module are executed by processor 2007, processor 2007 performs respective operations of the flow chart of FIG. 22.

FIG. 22 depicts an example of operations performed by a navigation system using 3D imaging information for a 3D anatomical volume to determine a position for a virtual implant.

At block 2210, processor 2007 may provide 3D imaging information for a 3D anatomical volume (e.g., the body 2150). At block 2220, processor 2007 may detect a pose of the probe 2110. The pose of the probe 2110 can include the position and/or the orientation of the probe 2110. In some embodiments, processor 2007 may detect the pose based on information received from a positioning system (also referred to as a tracking system). For example, the probe 2110 may include one or more spaced apart tracking markers 2111 and the positioning system may detect the position and orientation of the probe 2110 by detecting the tracking markers 2111. The processor 2007 can receive position data or orientation data from the positioning system via input interface 2001.

At block 2230, processor 2007 may determine a placement of the virtual implant 2120 for the 3D anatomical volume based on the pose of the probe 2110 and based on a default offset from an end of the probe 2110 along the longitudinal axis. The virtual implant 2120 can be overlaid on an image from the 3D anatomical volume such that the trajectory 2140 of the virtual implant 2120 is in alignment with the longitudinal axis of the probe in the orientation.

At block 2240, processor 2007 may provide reformatted image data via output interface 2003. The reformatted image data can be based on the 3D anatomical imaging information. In some embodiments, processor 2007 provides first reformatted image data to be rendered on a display (e.g., display 110) based on the 3D anatomical imaging information and based on the placement of the virtual implant. The 3-dimensional information can include the position and orientation or trajectory of the virtual implant 2120.

In additional or alternative embodiments, processor 2007 may provide new image data via output interface 2003. The new image data can be based on newly captured 3D anatomical imaging data and based on the placement of the virtual implant.

If processor 2007 has not received user input indicating a desire to adjust the virtual implant 2120, at block 2245 the processor 2007 may decide to repeat blocks 2220, 2230, and 2240. The image may be continuously updated and displayed on a display visible to a user such that the user can see changes in a position and trajectory of the virtual implant 2120 in response to movement of the probe 2110. If user input indicating a desire to adjust the virtual implant 2120 has been received, the processor 2007 may decide at block 2245 to proceed to block 2250. In some embodiments, processor 2007 can receive a signal from a button/pedal pressed by a user to indicate a desire to adjust the virtual implant 2120. In additional or alternative embodiments, processor 2007 can receive position data for the probe 2110 indicating the probe has moved in a predefined way so as to indicate a desire to adjust the virtual implant 2120. In additional or alternative embodiments, processor 2007 can receive user input from an input device (e.g., a keyboard or mouse) indicating a desire to adjust the virtual implant 2120.

At block 2250, processor 2007 adjusts the virtual implant 2120. In some embodiments, the processor 2007 adjusts the virtual implant 2120 after determining the placement of the virtual implant 2120 in block 2230 in response to movement of the probe 2110. The placement of the virtual implant 2120 can be adjusted while maintaining the trajectory 2140 of the virtual implant 2120. The processor 2007 may move the placement of the virtual implant 2120 along the trajectory 2140 responsive to movement of the probe 2110.

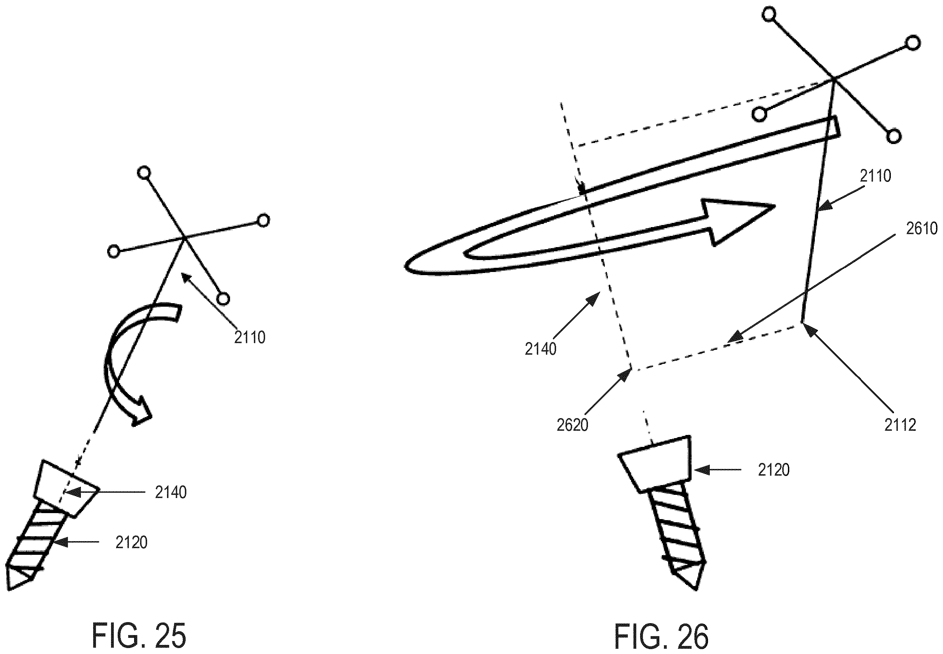

In some embodiments, processor 2007 can move the virtual implant 2120 in a first direction along the trajectory 2140 in response to detecting rotation of the probe 2110 in a first rotational direction and move the virtual implant 2120 in a second direction along the trajectory 2140 in response to detecting rotation of the probe in a second rotational direction. Such adjustment is discussed in greater detail with respect to FIG. 25.

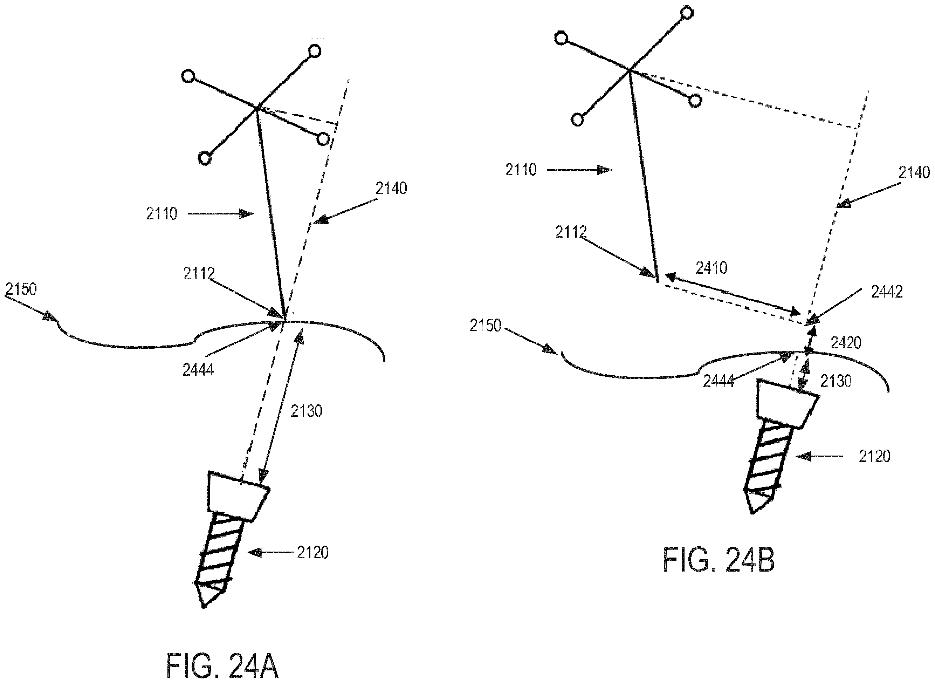

In additional or alternative embodiments, moving the virtual implant 2120 can include moving the virtual implant along the trajectory 2140 responsive to detecting movement of the tip 2112 of the probe 2110 in a direction that is nonparallel with respect to the trajectory 2140 of the virtual implant 2120. In additional or alternative embodiments, moving the virtual implant 2120 can include moving the virtual implant 2120 in a first direction along the trajectory 2140 in response to detecting movement of the tip 2112 of the probe 2110 away from the trajectory 2140 of the virtual implant 2120 and moving the virtual implant 2120 in a second direction along the trajectory 2140 in response to detecting movement of the tip 2112 of the probe 2110 toward the trajectory 2140. Such adjustment is discussed in greater detail below with respect to FIGS. 24A and 24B.

In additional or alternative embodiments, moving the virtual implant 2120 can include moving the virtual implant 2120 along the trajectory 2140 in response to detecting movement of the tip 2112 of the probe around the trajectory 2140 of the virtual implant. The virtual implant 2120 can be moved in a first direction along the trajectory in response to detecting movement of the tip 2112 of the probe 2110 in a clockwise direction around the trajectory 2140 and can be moved in a second direction along the trajectory 2140 in response to detecting movement of the tip 2112 of the probe 2110 in a counter-clockwise direction around the trajectory 2140. The scale of movement of the virtual implant 2120 along the trajectory 2140 can be relative to movement of the tip 2112 based on a distance of the tip from the trajectory 2140. Such adjustment is discussed in greater detail below with respect to FIG. 26.

FIGS. 24A-B depict an example of how a position of the tip 2112 of the probe 2110 can be used to adjust a position of the virtual implant 2120 along the trajectory 2140. In regards to FIG. 24A, processor 2007 can use position data of the tip 2112 of the probe 2110 to determine an initial position 2444 of the tip 2112, which may be on the trajectory 2140. An offset 2130 of the virtual implant 2120 from the initial position 2444 can be set to a default offset that extends into the 3D anatomical volume (body 2150 in this example). FIG. 24B depicts the probe 2110 in another position relative to the body 2150. As probe 2110 moves, processor 2007 can determine a closest corresponding point 2442 along the trajectory to the tip 2112. Processor 2007 can further determine a distance 2420 between the closest corresponding point 2442 and the initial position 2444 of the tip 2112. Distance 2420 can be used to set the offset 2130 of the virtual implant 2120, while maintaining the additional constraint that the virtual implant 2120 remain oriented along the trajectory 2140. In this example, as the closest point 2442 moves away from the position of the virtual implant 2120, the offset may be reduced, and as the closest point 2442 moves toward the position of the virtual implant 2120 the offset may be increased. The amount that the offset is adjusted may be proportional to the change in the distance 2420 between the closest point 2442 and the initial position 2444.

In additional or alternative embodiments, processor 2007 can adjust the offset 2130 by determining the distance 2410 between the initial position 2444 of the tip 2112 and the current location of the tip 2112. The closest point 2442 may remain the same while the offset is adjusted based on changes in the distance 2410. The offset 2130 can be based on a combination of distance 2420 and distance 2410.

FIG. 25 depicts an example of how a rotation of the probe 2110 about a longitudinal axis of the probe 2110 can be used to adjust a position of the virtual implant 2120 along the trajectory 2140. In some embodiments, processor 2007 can adjust a position of the virtual implant 2120 based on rotation of the probe 2110 without the probe 2110 moving along the trajectory 2140, which can be useful when the probe is bottomed on a pedicle or already in contact with a body. In some examples, processor 2007 can advance (e.g., move farther into a virtual body) a position of a virtual implant 2120 along a trajectory 2140 in response to detecting a clockwise rotation of the probe 2110 about the longitudinal axis of the probe 2110. The processor 2007 can retreat (e.g., move closer to a surface of the virtual body) a position of a virtual implant 2120 along a trajectory 2140 in response to detecting a counter-clockwise rotation of the probe 2110 about the longitudinal axis of the probe 2110. In additional or alternative embodiments, the speed of the movement or rotation of the probe 2110 can be determined by the probe 2110 and used to determine a direction that the virtual implant 2120 should be adjusted. For example, the probe 2110 may be quickly rotated counter-clockwise and then slowly rotated counter-clockwise and processor 2007 may detect this series of movements to indicate that the clockwise rotation should be used to increase the offset of the virtual implant 2120 and that the counter-clockwise rotation should be ignored (e.g., similar to a ratcheting action). In additional or alternative embodiments, the probe 2110 may be rendered as a virtual tap or other virtual surgical instrument or implant corresponding most closely to the implant-instrument combination that may be used in a subsequent surgery.

FIG. 26 depicts an example of how a rotation of the probe 2110 tip 2112 around the trajectory 2140 can be used to adjust a position of the virtual implant 2120 along the trajectory 2140. In some embodiments, the tip 2112 of the probe 2110 can be used to define a lever arm from the probe 2110 to a point 2620 on the trajectory 2140. Processor 2007 can determine a length of the lever arm based on a distance between the tip 2112 and the point 2620 and an angle of the lever arm based on the current orientation of the probe 2110 compared to a plane perpendicular to the trajectory 2140 that includes the point 2620. Processor 2007 can adjust the placement of the virtual implant 2120 based on detecting a user rotating or cranking the top of the probe 2110 about the trajectory 2140. The length and angle of the lever arm can affect the offset scaling. For example, lengthening the lever arm may reduce the amount of adjustment that is made to the position of the virtual implant 2120 per rotation of the probe 2110 about the trajectory 2140.

In some embodiments, processor 2007 can implement a ratcheting system such that adjustments to the position of the virtual implant 2120 are only made in response to rotation of the probe 2110 in one direction. For example, the processor 2007 may only adjust the offset of the virtual implant 2120 in response to detecting clockwise rotation of the probe 2110 allowing a user to adjust the virtual implant 2120 farther in a single direction along the trajectory 2140 while maintaining a small working envelope or with greater precision.

Any of the examples in FIGS. 24-26 can be combined to adjust the placement of the virtual implant 2120. In any of the embodiments, the ratio of user motion to offset adjustment can be scaled. The scaling may be informed by the position and orientation of the navigated instrument. For example, the processor 2007 can change the scaling when using a rotational mode based on changing the pitch of the probe 2110 or a distance between a point on the probe 2110 and a point on the trajectory 2140.

Returning to block 2250 of FIG. 22, in some embodiments, processor 2007 can adjust the virtual implant responsive to movement of the probe and responsive to user input separate from the movement of the probe while maintaining the trajectory of the virtual implant. For example, the user input may include depression of a button or pedal communicatively coupled to the navigation system.

In additional or alternative embodiments, adjusting the virtual implant 2120 can include changing a size of the virtual implant 2120 responsive to movement of the probe 2110 while maintaining the trajectory 2140 of the virtual implant as discussed above with respect to FIGS. 24-26. For example, processor 2007 can adjust a length or width of the virtual implant 2120 in response to rotation of the probe 2110. The virtual implant 2120 may be a virtual screw or any other suitable virtual implant.

At block 2260, processor 2007 provides the reformatted image data via output interface 2003. In some embodiments, processor 2007 provides second reformatted image data to be rendered on a display based on adjusting the virtual implant 2120. The second reformatted image data may be different than the first reformatted image data provided in block 2240. For example, since the trajectory 2140 is not changing, the second reformatted image data may not include trajectory data or else the transmitted trajectory data will remain constant while the virtual implant data changes. The first reformatted image data may not include virtual implant data or may include constant virtual implant data such as a default offset and default size or shape of the virtual implant 2120. In some embodiments, the first image may include a portion the body 2150 with a first marked location and first orientation of the virtual implant 2120 and the second image may include the portion of the body 2150 with a second marked location and second orientation of the virtual implant 2120. The second marked location may be shallower than the first marked location. The first image may further include a first depiction of the virtual implant 2120 as a particular type of implant with a particular size and the second image may include a second depiction of the virtual implant 2120 having a different type or a different size.

If processor 2007 has not received user input indicating a desire to store the virtual implant data, the processor 2007 may decide at block 2265 to repeat blocks 2250 and 2260. If processor 2007 receives user input indicating a desire to store the virtual implant data, the processor 2007 may determine at block 2265 to proceed to block 2270.

At block 2270, processor 2007 stores the virtual implant data. In some embodiments, the reformatted image data can be stored in response to user input. The reformatted image data may include a position, trajectory, size, shape, and type of the virtual implant 2120. In some examples, the virtual implant may be adjusted while a user depresses a button or a pedal (as discussed above) and the processor 2007 may store the information regarding the virtual implant in response to release of the button or pedal. In other embodiments, a first button/pedal may be used to initiate adjustment, and a second button/peal may be used to store the virtual implant data.

At block 2280, processor 2007 controls a robotic actuator. In some embodiments, processor 2007 can control a robotic actuator via control interface 2005 to position an end-effector based on the information regarding the virtual implant. For example, the processor 2007 can control a robotic actuator to position an end-effector such that a surgical operation can be performed using the end-effector to place a physical implant at a position in a body based on the information regarding the virtual implant 2120. In additional or alternative embodiments, processor 2007 can control the robotic actuator to perform the surgical operation and place a physical implant in the body based on the information regarding the virtual implant 2120.

In the above-description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belong. It will be further understood that terms, such as those defined in commonly used dictionaries, should be interpreted as having a meaning that is consistent with their meaning in the context of this specification and the relevant art and will not be interpreted in an idealized or overly formal sense unless expressly so defined herein.

When an element is referred to as being "connected", "coupled", "responsive", or variants thereof to another element, it can be directly connected, coupled, or responsive to the other element or intervening elements may be present. In contrast, when an element is referred to as being "directly connected", "directly coupled", "directly responsive", or variants thereof to another element, there are no intervening elements present. Like numbers refer to like elements throughout. Furthermore, "coupled", "connected", "responsive", or variants thereof as used herein may include wirelessly coupled, connected, or responsive. As used herein, the singular forms "a", "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus, a first element/operation in some embodiments could be termed a second element/operation in other embodiments without departing from the teachings of present inventive concepts. The same reference numerals or the same reference designators denote the same or similar elements throughout the specification.

As used herein, the terms "comprise", "comprising", "comprises", "include", "including", "includes", "have", "has", "having", or variants thereof are open-ended, and include one or more stated features, integers, elements, steps, components, or functions but does not preclude the presence or addition of one or more other features, integers, elements, steps, components, functions, or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

## Claims

1. A method of operating an image-guided surgical system using imaging information for a 3-dimensional anatomical volume, the method comprising:
detecting a pose of a probe (2110) based on information received from a tracking system (2111), wherein the probe (2110) defines a longitudinal axis and the probe is a physical device which is positioned adjacent to a body (2150);
generating an image of the body (2150) that includes a virtual implant (2120) using 3D anatomical image information,
displaying the image including the virtual implant (2120), a virtual implementation of the probe (2110) and a trajectory (2140) of the virtual implant (2120),
updating the image such that moving the probe (2110) adjacent to the body (2150) causes the virtual implementation of the probe, the trajectory of the virtual implant, or the virtual implant to move on the image displayed,
determining a placement of the virtual implant (2120) for the 3-dimensional anatomical volume based on the pose of the probe (2110) and based on an offset (2130) from an end of the probe along the longitudinal axis, such that the trajectory (2140) of the virtual implant (2120) is in alignment with the longitudinal axis of the probe (2110) in the pose;
and
after determining the placement of the virtual implant (2120), adjusting the virtual implant (2120) responsive to movement of the probe (2110) adjacent to the body (2150) while maintaining the trajectory (2140) of the virtual implant (2120),
wherein the determination of the placement of the virtual implant (2120) comprises to track the probe (2110), set the trajectory (2140) of the virtual implant responsive to a user input and determine the placement for the virtual implant (2120) at a default offset (2130) along the trajectory (2140),
and wherein adjusting the virtual implant (2120) comprises moving the placement of the virtual implant along the trajectory (2140) responsive to movement of the probe (2110).

2. The method of Claim 1 further comprising:
providing first reformatted image data to be rendered on a display (110; 304) based on the imaging information and based on the placement of the virtual implant, the imaging information including 3-dimensional anatomical imaging information for the 3-dimensional anatomical volume; and
after providing the first reformatted image data, providing second reformatted image data to be rendered on the display based on adjusting the virtual implant (2120).

3. The method of Claim 2, wherein adjusting comprises adjusting the virtual implant (2120) responsive to movement of the probe (2110) and responsive to user input separate from the movement of the probe while maintaining the trajectory (2140) of the virtual implant.

4. The method of Claim 3, wherein the user input is first user input, the method further comprising:
responsive to second user input separate from the movement of the probe (2110), storing information regarding the virtual implant (2120) based on the trajectory (2140) of the virtual implant and based on the adjusting.

5. The method of Claim 4, wherein moving the virtual implant (2120) comprises moving the virtual implant along the trajectory (2140) responsive to detecting rotation of the probe (2110) about the longitudinal axis of the probe.

6. The method of Claim 5, wherein moving the virtual implant (2120) comprising moving the virtual implant in a first direction along the trajectory (2140) responsive to detecting rotation of the probe (2110) in a first rotational direction and moving the virtual implant in a second direction along the trajectory responsive to detecting rotation of the probe in a second rotational direction.

7. The method of Claim 5, wherein moving the virtual implant (2120) comprises moving the virtual implant along the trajectory (2140) responsive to detecting rotation of the probe (2110) about the longitudinal axis of the probe in a first direction and maintaining a position of the virtual implant responsive to detecting rotation of the probe about the longitudinal axis of the probe in a second direction different than the first direction.

8. The method of Claim 4, wherein moving the virtual implant (2120) comprises moving the virtual implant along the trajectory (2140) responsive to detecting movement of a tip (2112) of the probe (2110) in a direction that is nonparallel with respect to the trajectory of the virtual implant.

9. The method of Claim 8, wherein moving the virtual implant (2120) comprises moving the virtual implant in a first direction along the trajectory (2140) responsive to detecting movement of the tip (2112) of the probe (2110) away from the trajectory of the virtual implant and moving the virtual implant in a second direction along the trajectory responsive to detecting movement of the tip of the probe toward the trajectory.

10. The method of Claim 4, wherein moving the virtual implant (2120) comprises moving the virtual implant along the trajectory (2140) responsive to detecting movement of a tip (2112) of the probe (2110) around the trajectory of the virtual implant.

11. The method of Claim 10 wherein moving the virtual implant (2120) comprises moving the virtual implant in a first direction along the trajectory (2140) responsive to detecting movement of the tip (2112) of the probe (2110) in a clockwise direction around the trajectory and moving the virtual implant in a second direction along the trajectory responsive to detecting movement of the tip of the probe in a counter clockwise direction around the trajectory.

12. The method of Claim 10, wherein a scale of movement of the virtual implant (2120) along the trajectory (2140) relative to movement of the tip (2112) is based on a distance of the tip from the trajectory.

13. The method of Claim 1, where adjusting the virtual implant (2120) comprises changing a size of the virtual implant responsive to movement of the probe (2010) while maintaining the trajectory (2140) of the virtual implant.

14. A surgical navigation system using imaging information for a 3-dimensional anatomical volume, the surgical navigation system comprising:
a display device (110; 304);
a probe (2110) which is a physical device ;
a tracking system comprising sensors (2160) and one or more tracking markers (2111) configured to track the movement of the probe (2110) in three dimensions;
a processor (2007); and
memory (2009) coupled with the processor (2007), wherein the memory (2009) comprises instructions stored therein, and wherein the instructions are executable by the processor to cause the processor (2007) to:
detect a three-dimensional pose of the probe (2110) adjacent to a body (2150) based on information received from a tracking system, wherein the probe (2110) defines a longitudinal axis;
generate an image of the body (2150) that includes a virtual implant (2120) using 3D anatomical image information,
display on the display device (110; 304) the image including the virtual implant (2120), a virtual implementation of the probe (2110) and a trajectory (2140) of the virtual implant (2120),
update the image such that moving the probe (2110) adjacent to the body (2150) causes the virtual implementation of the probe, the trajectory of the virtual implant, or the virtual implant to move on the image displayed, and
determine a placement of the virtual implant (2120) for the 3-dimensional anatomical volume based on the three-dimensional pose of the probe (2110) and based on an offset (2130) from an end of the probe along the longitudinal axis, such that the trajectory (2140) of the virtual implant (2120) is in alignment with the longitudinal axis of the probe (2110) in the three-dimensional pose;
after providing the placement of the virtual implant (2120), adjust the virtual implant (2120) responsive to movement of the probe (2110) adjacent to the body (2150) while maintaining the trajectory (2140) of the virtual implant (2120),
wherein the determination of the placement of the virtual implant (2120) comprises to track the probe (2110), set the trajectory (2140) of the virtual implant responsive to a user input and determine the placement for the virtual implant (2120) at a default offset (2130) along the trajectory (2140), and
and wherein adjusting the virtual implant (2120) comprises moving the placement of the virtual implant along the trajectory (2140) responsive to movement of the probe (2110).

15. The surgical navigation system of Claim 14, wherein the surgical navigation system is an image-guided surgical system and the imaging information is 3-dimensional anatomical imaging information, wherein the instructions are further executable by the processor (2007) for causing the processor to: provide first reformatted image data to be rendered on a display based on the 3-dimensional anatomical imaging information and based on the placement of the virtual implant (2120); and after providing the first reformatted image data, provide second reformatted image data to be rendered on the display based on adjusting the virtual implant (2120).

## Patentansprüche

1. Verfahren zum Betreiben eines bildgeführten chirurgischen Systems unter Verwendung von Bildgebungsinformationen für ein dreidimensionales anatomisches Volumen, wobei das Verfahren Folgendes umfasst:
Erkennen einer Position einer Sonde (2110) basierend auf Informationen, die von einem Tracking-System (2111) empfangen werden, wobei die Sonde (2110) eine Längsachse definiert und die Sonde eine physikalische Vorrichtung ist, die in der Nähe eines Körpers (2150) positioniert ist;
Erzeugen eines Bildes des Körpers (2150), das ein virtuelles Implantat (2120) enthält, unter Verwendung anatomischer 3D-Bildinformationen,
Anzeigen des Bildes, welches das virtuelle Implantat (2120), eine virtuelle Implementierung der Sonde (2110) und eine Bewegungsbahn (2140) des virtuellen Implantats (2120) umfasst,
Aktualisieren des Bildes, sodass eine Bewegung der Sonde (2110) in der Nähe des Körpers (2150) bewirkt, dass sich die virtuelle Implementierung der Sonde, die Bewegungsbahn des virtuellen Implantats oder das virtuelle Implantat auf dem angezeigten Bild bewegen,
Ermitteln einer Platzierung des virtuellen Implantats (2120) für das dreidimensionale anatomische Volumen basierend auf der Position der Sonde (2110) und basierend auf einem Versatz (2130) von einem Ende der Sonde entlang der Längsachse, sodass die Bewegungsbahn (2140) des virtuellen Implantats (2120) mit der Längsachse der Sonde (2110) in der Position fluchtet; und
nach der Ermittlung der Platzierung des virtuellen Implantats (2120), Justieren des virtuellen Implantats (2120) als Reaktion auf die Bewegung der Sonde (2110) in der Nähe des Körpers (2150) unter Beibehaltung der Bewegungsbahn (2140) des virtuellen Implantats (2120),
wobei die Ermittlung der Platzierung des virtuellen Implantats (2120) das Verfolgen der Sonde (2110), das Einstellen der Bewegungsbahn (2140) des virtuellen Implantats als Reaktion auf eine Benutzereingabe, und das Ermitteln der Platzierung für das virtuelle Implantat (2120) bei einem Standardversatz (2130) entlang der Bewegungsbahn (2140) umfasst,
und wobei das Justieren des virtuellen Implantats (2120) das Bewegen der Platzierung des virtuellen Implantats entlang der Bewegungsbahn (2140) als Reaktion auf die Bewegung der Sonde (2110) umfasst.

2. Verfahren nach Anspruch 1, ferner umfassend:
Bereitstellen erster neu formatierter Bilddaten, die auf einer Anzeige (110; 304) basierend auf den Bildgebungsinformationen und basierend auf der Platzierung des virtuellen Implantats wiedergegeben werden sollen, wobei die Bildgebungsinformationen dreidimensionale anatomische Bildgebungsinformationen für das dreidimensionale anatomische Volumen umfassen; und
nach dem Bereitstellen der ersten neu formatierter Bilddaten, Bereitstellen zweiter neu formatierter Bilddaten, die auf der Anzeige basierend auf der Justierung des virtuellen Implantats (2120) wiedergegeben werden sollen.

3. Verfahren nach Anspruch 2, wobei das Justieren das Anpassen des virtuellen Implantats (2120) als Reaktion auf die Bewegung der Sonde (2110) und als Reaktion auf eine von der Bewegung der Sonde getrennte Benutzereingabe unter Beibehaltung der Bewegungsbahn (2140) des virtuellen Implantats (2120) umfasst,

4. Verfahren nach Anspruch 3, wobei die Benutzereingabe eine erste Benutzereingabe ist, wobei das Verfahren ferner umfasst:
Speichern von Informationen bezüglich des virtuellen Implantats (2120) basierend auf der Bewegungsbahn (2140) des virtuellen Implantats und basierend auf der Justierung als Reaktion auf eine zweite, von der Bewegung der Sonde (2110) getrennte Benutzereingabe.

5. Verfahren nach Anspruch 4, wobei das Bewegen des virtuellen Implantats (2120) das Bewegen des virtuellen Implantats entlang der Bewegungsbahn (2140) als Reaktion auf das Erfassen einer Drehung der Sonde (2110) um die Längsachse der Sonde umfasst.

6. Verfahren nach Anspruch 5, wobei das Bewegen des virtuellen Implantats (2120) das Bewegen des virtuellen Implantats in einer ersten Richtung entlang der Bewegungsbahn (2140) als Reaktion auf das Erfassen einer Drehung der Sonde (2110) in einer ersten Drehrichtung, und das Bewegen des virtuellen Implantats in einer zweiten Richtung entlang der Bewegungsbahn als Reaktion auf das Erfassen einer Drehung der Sonde in einer zweiten Drehrichtung umfasst.

7. Verfahren nach Anspruch 5, wobei das Bewegen des virtuellen Implantats (2120) das Bewegen des virtuellen Implantats entlang der Bewegungsbahn (2140) als Reaktion auf das Erfassen einer Drehung der Sonde (2110) um die Längsachse der Sonde in einer ersten Richtung, und das Beibehalten einer Position des virtuellen Implantats als Reaktion auf das Erfassen einer Drehung der Sonde um die Längsachse der Sonde in einer zweiten Richtung umfasst, die sich von der ersten Richtung unterscheidet.

8. Verfahren nach Anspruch 4, wobei das Bewegen des virtuellen Implantats (2120) das Bewegen des virtuellen Implantats entlang der Bewegungsbahn (2140) in Reaktion auf das Erfassen einer Bewegung einer Spitze (2112) der Sonde (2110) in einer Richtung umfasst, die in Bezug auf die Bewegungsbahn des virtuellen Implantats nicht parallel ist.

9. Verfahren nach Anspruch 8, wobei das Bewegen des virtuellen Implantats (2120) das Bewegen des virtuellen Implantats in einer ersten Richtung entlang der Bewegungsbahn (2140) als Reaktion auf das Erfassen einer Bewegung der Spitze (2112) der Sonde (2110) weg von der Bewegungsbahn des virtuellen Implantats, und das Bewegen des virtuellen Implantats in einer zweiten Richtung entlang der Bewegungsbahn als Reaktion auf das Erfassen einer Bewegung der Spitze der Sonde in Richtung der Bewegungsbahn umfasst.

10. Verfahren nach Anspruch 4, wobei das Bewegen des virtuellen Implantats (2120) das Bewegen des virtuellen Implantats entlang der Bewegungsbahn (2140) als Reaktion auf das Erfassen einer Bewegung einer Spitze (2112) der Sonde (2110) um die Bewegungsbahn des virtuellen Implantats umfasst.

11. Verfahren nach Anspruch 10, wobei das Bewegen des virtuellen Implantats (2120) das Bewegen des virtuellen Implantats in einer ersten Richtung entlang der Bewegungsbahn (2140) als Reaktion auf das Erfassen einer Bewegung der Spitze (2112) der Sonde (2110) im Uhrzeigersinn um die Bewegungsbahn, und das Bewegen des virtuellen Implantats in einer zweiten Richtung entlang der Bewegungsbahn als Reaktion auf das Erfassen einer Bewegung der Spitze der Sonde gegen den Uhrzeigersinn um die Bewegungsbahn umfasst.

12. Verfahren nach Anspruch 10, wobei Bewegungsumfang des virtuellen Implantats (2120) entlang der Bewegungsbahn (2140) relativ zur Bewegung der Spitze (2112) auf einem Abstand der Spitze von der Bewegungsbahn basiert.

13. Verfahren nach Anspruch 1, wobei das Justieren des virtuellen Implantats (2120) das Ändern einer Größe des virtuellen Implantats als Reaktion auf die Bewegung der Sonde (2010) unter Beibehaltung der Bewegungsbahn (2140) des virtuellen Implantats umfasst.

14. Chirurgisches Navigationssystem, das Bildgebungsinformationen für ein dreidimensionales anatomisches Volumen verwendet, wobei das chirurgische Navigationssystem Folgendes umfasst:
eine Anzeigevorrichtung (110; 304);
eine Sonde (2110), die eine physikalische Vorrichtung ist;
ein Tracking-System, das Sensoren (2160) und einen oder mehrere Trackingmarker (2111) umfasst, die zum Verfolgen der Bewegung der Sonde (2110) in drei Dimensionen konfiguriert sind;
einen Prozessor (2007); und
einen Speicher (2009), der mit dem Prozessor (2007) verbunden ist, wobei der Speicher (2009) darin gespeicherte Anweisungen umfasst, und wobei die Anweisungen durch den Prozessor ausführbar sind, damit der Prozessor (2007) Folgendes bewirkt:
Erfassen einer dreidimensionalen Position der Sonde (2110) in der Nähe eines Körpers (2150) basierend auf Informationen, die von einem Tracking-System empfangen werden, wobei die Sonde (2110) eine Längsachse definiert;
Erzeugen eines Bildes des Körpers (2150), das ein virtuelles Implantat (2120) umfasst, unter Verwendung anatomischer 3D-Bildinformationen,
Anzeigen auf der Anzeigevorrichtung (110; 304) das Bild, welches das virtuelle Implantat (2120), eine virtuelle Implementierung der Sonde (2110) und eine Bewegungsbahn (2140) des virtuellen Implantats (2120) umfasst,
Aktualisieren des Bildes, sodass eine Bewegung der Sonde (2110) in der Nähe des Körpers (2150) eine Bewegung der virtuellen Implementierung der Sonde, der Bewegungsbahn des virtuellen Implantats oder des virtuellen Implantats auf dem angezeigten Bild bewirkt, und
Ermitteln einer Platzierung des virtuellen Implantats (2120) für das dreidimensionale anatomische Volumen basierend auf der dreidimensionalen Position der Sonde (2110) und basierend auf einem Versatz (2130) von einem Ende der Sonde entlang der Längsachse, sodass die Bewegungsbahn (2140) des virtuellen Implantats (2120) mit der Längsachse der Sonde (2110) in der dreidimensionalen Position fluchtet;
nach der Bereitstellung der Platzierung des virtuellen Implantats (2120), Justieren des virtuellen Implantats (2120) als Reaktion auf eine Bewegung der Sonde (2110) in der Nähe des Körpers (2150) unter Beibehaltung der Bewegungsbahn (2140) des virtuellen Implantats (2120),
wobei die Ermittlung der Platzierung des virtuellen Implantats (2120) das Verfolgen der Sonde (2110), das Einstellen der Bewegungsbahn (2140) des virtuellen Implantats als Reaktion auf eine Benutzereingabe und das Ermitteln der Platzierung für das virtuelle Implantat (2120) bei einem Standardversatz (2130) entlang der Bewegungsbahn (2140) umfasst,
und wobei das Justieren des virtuellen Implantats (2120) das Bewegen der Platzierung des virtuellen Implantats entlang der Bewegungsbahn (2140) als Reaktion auf die Bewegung der Sonde (2110) umfasst.

15. Chirurgisches Navigationssystem nach Anspruch 14, wobei das chirurgische Navigationssystem ein bildgeführtes chirurgisches System ist und die Bildgebungsinformationen dreidimensionale anatomische Bildgebungsinformationen sind, wobei die Befehle ferner durch den Prozessor (2007) ausführbar sind, damit der Prozessor (2007) Folgendes bewirkt: Bereitstellen erster neu formatierter Bilddaten, die auf einer Anzeige basierend auf den dreidimensionalen anatomischen Bildgebungsinformationen und basierend auf der Platzierung des virtuellen Implantats (2120) wiedergegeben werden sollen; und nach dem Bereitstellen der ersten umformatierten Bilddaten, Bereitstellen zweiter neu formatierter Bilddaten, die auf der Anzeige basierend auf der Einstellung des virtuellen Implantats (2120) wiedergegeben werden sollen.

## Revendications

1. Procédé pour faire fonctionner un système chirurgical guidé par image utilisant des informations d'imagerie pour un volume anatomique tridimensionnel, le procédé comprenant:
la détection d'une pose d'une sonde (2110) sur la base d'informations reçues d'un système de suivi (2111), dans lequel la sonde (2110) définit un axe longitudinal et la sonde est un dispositif physique qui est positionné de manière adjacente à un corps (2150);
la formation d'une image du corps (2150) qui inclut un implant virtuel (2120) à l'aide d'informations d'image anatomique 3D,
l'affichage de l'image incluant l'implant virtuel (2120), une mise en oeuvre virtuelle de la sonde (2110) et une trajectoire (2140) de l'implant virtuel (2120),
la mise à jour de l'image de telle sorte que le déplacement de la sonde (2110) de manière adjacente au corps (2150) amène la mise en oeuvre virtuelle de la sonde, la trajectoire de l'implant virtuel, ou l'implant virtuel à se déplacer sur l'image affichée,
la détermination d'un emplacement de l'implant virtuel (2120) pour le volume anatomique tridimensionnel sur la base de la pose de la sonde (2110) et sur la base d'un décalage (2130) par rapport à une extrémité de la sonde le long de l'axe longitudinal, de telle sorte que la trajectoire (2140) de l'implant virtuel (2120) est en alignement avec l'axe longitudinal de la sonde (2110) dans la pose; et
après la détermination de l'emplacement de l'implant virtuel (2120), l'ajustement de l'implant virtuel (2120) en réponse au déplacement de la sonde (2110) de manière adjacente au corps (2150) tout en maintenant la trajectoire (2140) de l'implant virtuel (2120),
dans lequel la détermination de l'emplacement de l'implant virtuel (2120) comprend le suivi de la sonde (2110), le réglage de la trajectoire (2140) de l'implant virtuel en réponse à une entrée utilisateur et la détermination de l'emplacement de l'implant virtuel (2120) à un décalage par défaut (2130) le long de la trajectoire (2140),
et dans lequel l'ajustement de l'implant virtuel (2120) comprend le déplacement de l'emplacement de l'implant virtuel le long de la trajectoire (2140) en réponse au déplacement de la sonde (2110).

2. Procédé selon la revendication 1 comprenant en outre:
la fourniture de premières données d'image reformatées destinées à être restituées sur un affichage (110; 304) sur la base des informations d'imagerie et sur la base de l'emplacement de l'implant virtuel, les informations d'imagerie incluant des informations d'imagerie anatomique tridimensionnelle pour le volume anatomique tridimensionnel; et
après la fourniture des premières données d'image reformatées, la fourniture de secondes données d'image reformatées destinées à être restituées sur l'affichage sur la base de l'ajustement de l'implant virtuel (2120).

3. Procédé selon la revendication 2, dans lequel l'ajustement comprend l'ajustement de l'implant virtuel (2120) en réponse au déplacement de la sonde (2110) et en réponse à une entrée utilisateur séparée du déplacement de la sonde tout en maintenant la trajectoire (2140) de l'implant virtuel.

4. Procédé selon la revendication 3, dans lequel l'entrée utilisateur est une première entrée utilisateur, le procédé comprenant en outre:
en réponse à une seconde entrée utilisateur séparée du déplacement de la sonde (2110), le stockage d'informations concernant l'implant virtuel (2120) sur la base de la trajectoire (2140) de l'implant virtuel et sur la base de l'ajustement.

5. Procédé selon la revendication 4, dans lequel le déplacement de l'implant virtuel (2120) comprend le déplacement de l'implant virtuel le long de la trajectoire (2140) en réponse à la détection de la rotation de la sonde (2110) autour de l'axe longitudinal de la sonde.

6. Procédé selon la revendication 5, dans lequel le déplacement de l'implant virtuel (2120) comprenant le déplacement de l'implant virtuel dans une première direction le long de la trajectoire (2140) en réponse à la détection de la rotation de la sonde (2110) dans un premier sens de rotation et le déplacement de l'implant virtuel dans une seconde direction le long de la trajectoire en réponse à la détection de la rotation de la sonde dans un second sens de rotation.

7. Procédé selon la revendication 5, dans lequel le déplacement de l'implant virtuel (2120) comprend le déplacement de l'implant virtuel le long de la trajectoire (2140) en réponse à la détection de la rotation de la sonde (2110) autour de l'axe longitudinal de la sonde dans une première direction et le maintien d'une position de l'implant virtuel en réponse à la détection de la rotation de la sonde autour de l'axe longitudinal de la sonde dans une seconde direction différente de la première direction.

8. Procédé selon la revendication 4, dans lequel le déplacement de l'implant virtuel (2120) comprend le déplacement de l'implant virtuel le long de la trajectoire (2140) en réponse à la détection du déplacement d'une pointe (2112) de la sonde (2110) dans une direction qui n'est pas parallèle à la trajectoire de l'implant virtuel.

9. Procédé selon la revendication 8, dans lequel le déplacement de l'implant virtuel (2120) comprend le déplacement de l'implant virtuel dans une première direction le long de la trajectoire (2140) en réponse à la détection du déplacement de la pointe (2112) de la sonde (2110) en s'écartant de la trajectoire de l'implant virtuel et le déplacement de l'implant virtuel dans une seconde direction le long de la trajectoire en réponse à la détection du déplacement de la pointe de la sonde vers la trajectoire.

10. Procédé selon la revendication 4, dans lequel le déplacement de l'implant virtuel (2120) comprend le déplacement de l'implant virtuel le long de la trajectoire (2140) en réponse à la détection du déplacement d'une pointe (2112) de la sonde (2110) autour de la trajectoire de l'implant virtuel.

11. Procédé selon la revendication 10, dans lequel le déplacement de l'implant virtuel (2120) comprend le déplacement de l'implant virtuel dans une première direction le long de la trajectoire (2140) en réponse à la détection du déplacement de la pointe (2112) de la sonde (2110) dans le sens des aiguilles d'une montre autour de la trajectoire et le déplacement de l'implant virtuel dans une seconde direction le long de la trajectoire en réponse à la détection du déplacement de la pointe de la sonde dans le sens inverse des aiguilles d'une montre autour de la trajectoire.

12. Procédé selon la revendication 10, dans lequel une échelle de déplacement de l'implant virtuel (2120) le long de la trajectoire (2140) par rapport au déplacement de la pointe (2112) est basée sur une distance de la pointe par rapport à la trajectoire.

13. Procédé selon la revendication 1, dans lequel l'ajustement de l'implant virtuel (2120) comprend la modification d'une taille de l'implant virtuel en réponse au déplacement de la sonde (2010) tout en maintenant la trajectoire (2140) de l'implant virtuel.

14. Système de navigation chirurgicale utilisant des informations d'imagerie pour un volume anatomique tridimensionnel, le système de navigation chirurgicale comprenant:
un dispositif d'affichage (110; 304);
une sonde (2110) qui est un dispositif physique;
un système de suivi comprenant des capteurs (2160) et un ou plusieurs marqueurs de suivi (2111) configurés pour suivre le déplacement de la sonde (2110) en trois dimensions;
un processeur (2007); et
une mémoire (2009) couplée au processeur (2007), dans lequel la mémoire (2009) comprend des instructions qui y sont stockées, et dans lequel les instructions sont exécutables par le processeur pour amener le processeur (2007) à:
détecter une pose tridimensionnelle de la sonde (2110) de manière adjacente à un corps (2150) sur la base d'informations reçues d'un système de suivi, dans lequel la sonde (2110) définit un axe longitudinal;
former une image du corps (2150) qui inclut un implant virtuel (2120) à l'aide d'informations d'image anatomique 3D,
afficher sur le dispositif d'affichage (110; 304) l'image incluant l'implant virtuel (2120), une mise en oeuvre virtuelle de la sonde (2110) et une trajectoire (2140) de l'implant virtuel (2120),
mettre à jour l'image de telle sorte que le déplacement de la sonde (2110) de manière adjacente au corps (2150) amène la mise en oeuvre virtuelle de la sonde, la trajectoire de l'implant virtuel ou l'implant virtuel à se déplacer sur l'image affichée, et
déterminer un emplacement de l'implant virtuel (2120) pour le volume anatomique tridimensionnel sur la base de la pose tridimensionnelle de la sonde (2110) et sur la base d'un décalage (2130) par rapport à une extrémité de la sonde le long de l'axe longitudinal, de telle sorte que la trajectoire (2140) de l'implant virtuel (2120) est en alignement avec l'axe longitudinal de la sonde (2110) dans la pose tridimensionnelle;
après la fourniture de l'emplacement de l'implant virtuel (2120), ajuster l'implant virtuel (2120) en réponse au déplacement de la sonde (2110) de manière adjacente au corps (2150) tout en maintenant la trajectoire (2140) de l'implant virtuel (2120),
dans lequel la détermination de l'emplacement de l'implant virtuel (2120) comprend le suivi de la sonde (2110), le réglage de la trajectoire (2140) de l'implant virtuel en réponse à une entrée utilisateur et la détermination de l'emplacement pour l'implant virtuel (2120) à un décalage par défaut (2130) le long de la trajectoire (2140), et
et dans lequel l'ajustement de l'implant virtuel (2120) comprend le déplacement de l'emplacement de l'implant virtuel le long de la trajectoire (2140) en réponse au déplacement de la sonde (2110).

15. Système de navigation chirurgicale selon la revendication 14, dans lequel le système de navigation chirurgicale est un système chirurgical guidé par image et les informations d'imagerie sont des informations d'imagerie anatomique tridimensionnelle, dans lequel les instructions sont en outre exécutables par le processeur (2007) pour amener le processeur à: fournir des premières données d'image reformatées destinées à être restituées sur un affichage sur la base des informations d'imagerie anatomique tridimensionnelle et sur la base de l'emplacement de l'implant virtuel (2120); et après la fourniture des premières données d'image reformatées, fournir des secondes données d'image reformatées destinées à être restituées sur l'affichage sur la base de l'ajustement de l'implant virtuel (2120).
